(19) Europäisches Patentamt
European Patent Office
Office européen des brevets

(11) EP 0 500 137 B1

(12) EUROPEAN PATENT SPECIFICATION

(45) Date of publication and mention of the grant of the patent:
**15.05.1996 Bulletin 1996/20**

(21) Application number: **92102982.3**

(22) Date of filing: **24.02.1992**

(51) Int Cl.6: **C07D 487/04**, C07F 7/18, C07D 471/14, C07D 495/14, A61K 31/505
// (C07D487/04, 249:00, 239:00), (C07D495/14, 335:00, 249:00, 239:00)

# (54) 1,2,4-Triazolo[1,5-a]pyrimidine derivatives, a process for preparing them as well as medicaments containing them and the use of them

1,2,4-Triazolo[1,5-a]pyrimidinderivate, Verfahren zu ihrer Herstellung, diese enthaltende Arzneimittel und ihre Verwendung

Dérivés de la 1,2,4-triazolo[1,5-a]pyrimidine, procédé de préparation, médicaments les contenant et leur utilisation

(84) Designated Contracting States:
**AT BE CH DE DK ES FR GB GR IT LI LU MC NL PT SE**

(30) Priority: **22.02.1991 HU 58891**

(43) Date of publication of application:
**26.08.1992 Bulletin 1992/35**

(73) Proprietor: **EGIS GYOGYSZERGYAR**
**H-1106 Budapest (HU)**

(72) Inventors:
- **Berecz, Gábor**
  **H-2750 Nagykorös (HU)**
- **Reiter, Jozsef, Dr.**
  **H-1022 Budapest (HU)**
- **Reiter born Esses, Klára, Dr.**
  **H-1022 Budapest (HU)**
- **Pongo, Lászlo, Dr.**
  **H-1161 Budapest (HU)**
- **Rivo, Endre, Dr.**
  **H-1126 Budapest (HU)**
- **Trinka, Péter**
  **H-1116 Budapest (HU)**

(74) Representative: **Beszédes, Stephan G., Dr.**
**Patentanwalt**
**Postfach 11 68**
**D-85201 Dachau (DE)**

(56) References cited:

| | |
|---|---|
| **EP-A- 0 150 507** | **DD-A- 99 794** |
| **DE-A- 3 343 360** | **GB-A- 1 423 266** |
| **US-A- 2 933 388** | |

- **JOURNAL OF HETEROCYCLIC CHEMISTRY. vol. 27, 1990, PROVO US pages 1559 - 1563; R. L. TOLMAN ET AL.: 'Annelated piperazinyl-7,8-dihydro-6H-thiopyrano(3,2-d)pyrimidines'**
- **SYNLETT no. 2, February 1991, SUTTGART, DE pages 115 - 116; K. MINN: 'Chalcones via a palladium-catalyzed coupling of iodoheterocycles to 1-phenyl-2-propyn-1-ol'**
- **CHEMICAL AND PHARMACEUTICAL BULLETIN vol. 8, 1960, TOKYO, JP pages 162 - 167; T. OKABAYASHI: 'Action of substituted azaindolizines on microorganisms'**
- **INDIAN JOURNAL OF CHEMISTRY vol. 28B, no. 3, 1989, NEW DELHI, INDIA pages 242 - 246; VISHNU J. RAM: 'Chemotherapeutic agents : part XIII - Synthesis of 2-pyridyl-1,2,4-triazolo(1,5-a)pyrimidines as antimicrobial agent'**
- **CHEMICAL ABSTRACTS, vol. 110, 1989, Columbus, Ohio, US; abstract no. 212752M, VISHNU J. RAM: 'Chemotherapeutic agents. Part X. Synthesis of 2-pyridyl(1,2,4)triazolo(1,5-a)pyrimidines as leishmanicides' page 747; & Indian J. Chem., Sect. B 1988, 27B(9), 825-9**

EP 0 500 137 B1

- **JOURNAL OF ORGANIC CHEMISTRY. vol. 39, no. 9, 1974, EASTON US pages 1256 - 62; R. L. TOLMAN: 's-Triazolo(1,5-a)pyrimidine nucleosides. Site of N-glycosylation studies and the synthesis of an N-bridgehead guanosine analog'**
- **CHEMICAL ABSTRACTS, vol. 105, 1986, Columbus, Ohio, US; abstract no. 226617E, K. HIRAI: 'Pyrazolo(1,5-a)- and (1,2,4)triazolo(1,5-a)pyrimidine derivatives' page 797 ;**
- **CHEMICAL ABSTRACTS, vol. 105, 1986, Columbus, Ohio, US; abstract no. 97493K, 'Imidazopyrimidines and analogs' page 640 ; & jp 61 56180, Fujiasawa**


Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The invention is concerned with novel 1,2,4-triazo-lo[1,5-a]pyrimidine derivatives of the general formula

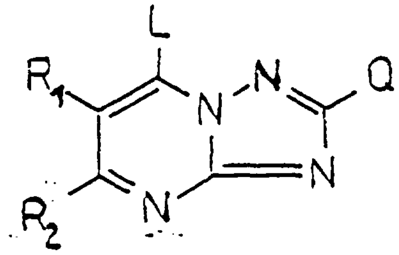

I

with the definitions indicated below including their salts, a process for preparing them as well as medicaments containing these compounds and the use of them for preparing medicaments or as intermediates for preparing the 1,2,4-triazolo-[1,5-a]pyrimidine derivatives of the general formula

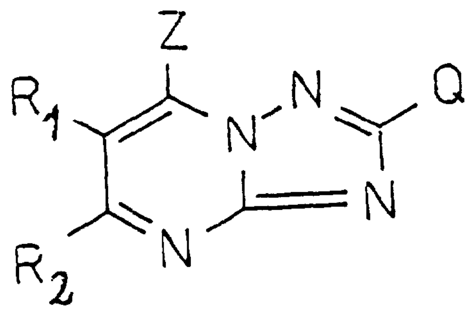

VII

with the definitions indicated below.

From US Patent 2,933,388 compounds have been known, in which

- Q stands for hydrogen,
- $R_1$ means an isobutyl radical,
- $R_2$ represents a methyl radical and
- L stands for chlorine.

As application only the further reaction with thiourea to the corresponding thiols for use only in photographic treating baths has been indicated, no mention having been made of the use in medicaments nor of that serving as intermediates for the preparation of 1,2,4-triazolo[1,5-a]pyrimidine derivatives of the general formula

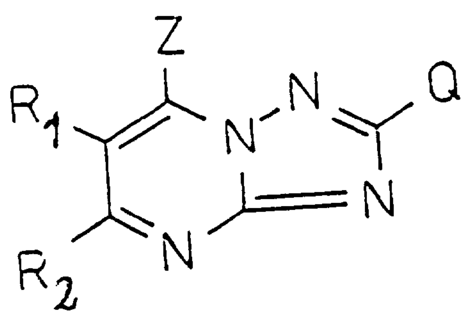

VII

In Journal of Heterocyclic Chemistry, Vol. 27, 1990, pages 1 559 to 1 563 on page 1 563 a compound 12 has been described, in which

- Q stands for hydrogen,
- L means chlorine and
- $R_1$ and
- $R_2$ together represent the formula C with

  Y = sulphur,

n = 0 and

m = 3.

This compound is used only for preparing optionally substituted piperazinyl derivatives of it. Though at the beginning of the said reference globally antidiabetic properties have been indicated it is questionable if even this is to be construed as applying for the said piperazine derivatives.

The publication SYNLETT, No. 2, February 1991, pages 115 to 116 includes a compound 6d in which

Q stands for hydrogen,
L means iodine,
$R_1$ represents hydrogen and
$R_2$ stands for a methyl radical.

The only application indicated is to prepare from them the corresponding carbinols no mention having been made of the use in medicaments.

In Chemical and Pharmaceutical Bulletin, Vol. 8, 1960, pages 162 to 167 on page 164, table IV, lines 5 to 7 there have been described compounds, in which

Q stands for hydrogen,
L means chlorine,
$R_1$ represents hydrogen or an ethyl group and
$R_2$ stands for hydrogen or a methyl group.

As application it has been indicated the inhibition of the growth of Escherichia coli in a salt-glucose-medium.

From Indian Journal of Chemistry, Vol. 288, No. 3, 1989, pages 242 to 246, particularly page 244, Example 7b there has been known a compound, in which

Q stands for a 3-pyridyl radical,
L means chlorine,
$R_1$ represents hydrogen and
$R_2$ stands for a methyl radical.

The activity as an antimicrobial agent has been examined. However, according to page 244, lines 5 to 6 none of the compounds showed any worth mentioning antimicrobial activity. Furthermore there it has been indicated that this compound can be further reacted with an amine, hydrazine, thiourea or ethyl thioglycolate the lack of activity also applying to the compounds so prepared.

In Chemical Abstracts, Vol. 110, 1989, Abstract No. 212752m, page 747 it has been described a compound, in which

Q stands for a 2- or 4-pyridyl radical,
L means chlorine,
$R_1$ represents hydrogen and
$R_2$ stands for a methyl group.

These compounds serve only as intermediates for being reacted with 4-methylpiperazine or hydrazine or thiourea or for being condensed with acetylacetone, ethoxymethylenemalononitrile, ethyl ethoxymethylenecyanoacetateor di-(ethyl)-ethoxymethylenemalonate to obtain compounds useful as chemotherapeutic agents. No therapeutic use of the said intermediates themselves has been indicated.

From European "Offenlegungsschrift" 150 507, particularly page 105, line 10 to page 106, line 5 there has been known a compound, in which

Q represent formula B
with $R_4$ = $R_5$ = hydrogen,
L stands for chlorine,
$R_1$ means hydrogen and
$R_2$ represents a methyl radical.

No use of this compound in medicaments has been indicated it serving only as intermediate for preparing (6R,7R)-7-[2-(2--amino-4-thiazol-yl)-2-(Z-methoxyimino)acetamido]-3-[(2-amino-5-methyl-s-triazolo[1,5-a]pyrimidine-7-yl)thiomethyl]-8--oxo-5-thia-1-azabicyclo[4.2.0]oct-2-ene-2-carboxylic acid via 2-amino-7-mercapto-5-methyl-s-triazolo [1,5-a]pyrimidine. As application for such end product the treatment and/or the prevention of infectious diseases has been indicated.

From German "Offenlegungsschrift" 33 43 360, particularly page 19, lines 10 to 22, it has been known a compound, in which

Q stands for hydrogen,
L means chlorine,
$R_1$ represents hydrogen and
$R_2$ stands for a isobutyl radical.

For this compound no mention has been made of its use in medicaments. Only its quality of being an intermediate for preparing 4-mercapto-6-tert.butyl-1,3,3a,7-tetraazainden which itself is used in the photographic field has been indicated.

In Ex-DDR Patent DD 99 794, particularly in Examples 1 and 6 compounds have been described, in which

Q stands for hydrogen,
L means chlorine,
$R_1$ represents hydrogen or a methyl radical and
$R_2$ represents hydrogen.

For these compounds no use in medicaments has been mentioned. It has been only indicated that they serve as intermediates for being reacted with amines to yield compounds having coronary dilating activity.

From British Patent 1,423,266, particularly page 12, a compound 25, in which

Q represents formula A with

p = O
$R_3$ = benzyl radical,

L means chlorine,

$R_1$ represents hydrogen and

$R_2$ stands for a methyl radical

has been known.

For this compound by the heading of table II on page 12 the application "as 3',5'-cyclic AMP phosphordiesterase inhibitor" has been indicated. Moreover it can serve as intermediate for being reacted with an amine, hydrazine or sodium hydrosulfide to give compounds with the same activity and some of them also a coronary dilating activity.

In Journal of Organic Chemistry, Vol. 39, No. 9, 1974, Easton, USA, pages 1 256 to 1 262, particularly page 1 257, compound No. 2 it has been described a compound, in which

Q stands for hydrogen,
L means a trimethylsilyloxy group,
$R_1$ represents hydrogen and
$R_2$ stands for a methyl radical.

No use of this compound in medicaments has been indicated. Rather it serves as an intermediate for being glycosylated no application of the so prepared end products having been indicated.

From Chemical Abstracts, Vol. 105, 1986, Abstract No. 226617e, page 797, it has been known a compound, in which

Q stands for hydrogen,

L means chlorine,
$R_1$ represents a methyl radical and
$R_2$ stands for a methyl radical.

No mention of a use of this compound in medicaments has been mentioned. It serves only as an intermediate for being reacted with amine hydrochlorides or amide hydrochlorides to yield compounds having antiulcer activity.

From Chemical Abstracts, Vol. 105, 1986, Abstract No. 97493k, page 640, there have been known imidazopyrimidines and analogs from which one showed an inhibition of ulcer in mice. No compound of formula I has been described the structure of these imidazopyrimidines being considerably different from the 1,2,4-triazolo[1,5-a]pyrimidine derivatives of the general formula I. Thus first of all the presence of an imidazole ring instead of a triazole ring is a fundamental difference. As far as $R^4$ in the compounds of the best-mentioned reference stands for a pyridine group this cannot be bound to the imidazole ring by its nitrogen atom contrary to the heterocyclic group of 1,2,4-triazolo[1,5-a]pyrimidine derivatives of the general formula I which anyhow can have only other heterocyclic groups on the triazole ring.

The problem underlying to the invention is to create novel 1,2,4-triazolo[1,5-a]pyrimidine derivatives showing valuable pharmacological properties, particularly gastric acid secretion inhibiting effect, a process for preparing them as well as medicaments containing these compounds and the use of them.

Surprisingly this has been solved by the invention.

Thus a subject-matter of the invention are 1,2,4-triazolo[1,5-a]pyrimidine derivatives of the general formula

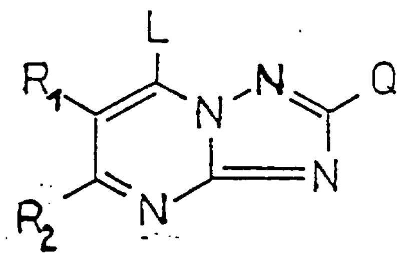

I

wherein

Q represents a morpholino, piperazino or piperidinyl group bound by a nitrogen atom of them to the triazole ring, optionally carrying a $C_{1-4}$ alkyl or benzyl substituent; or a group of the formula

$$S(O)_p\text{-}R_3 \qquad A,$$

wherein

- p stands for 0, 1 or 2 and
- $R_3$ denotes straight or branched chained $C_{1-8}$ alkyl, $C_{2-6}$ alkenyl or phenyl-($C_{1-4}$ alkyl), which latter may optionally carry one or more halogen or nitro substituent(s), or phenyl, optionally substituted by one or more nitro and/or trifluoromethyl;
  or a group of the formula

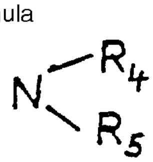

B ,

wherein

- $R_4$ and $R_5$ independently each represent hydrogen, straight or branched chained $C_{1-12}$ alkyl, $C_{2-6}$ alkenyl, phenyl--($C_{1-4}$ alkyl) including such having more than 1 phenyl group or di-($C_{1-4}$ alkyl)--amino-($C_{1-6}$ alkyl),

$R_1$ and $R_2$ independently each represent hydrogen or straight or branched chained $C_{1-4}$ alkyl or
$R_1$ and $R_2$ together form a group of the formula

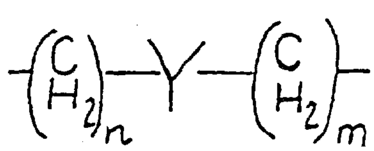

C,

in which latter

Y stands for a $CH_2$ group, a sulfur atom or a group of the formula

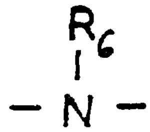 D,

wherein

$R_6$ denotes hydrogen or phenyl-($C_{1-4}$ alkyl),

n and m each represent 0, 1, 2, 3, 4 or 5

and

L represents halogen,

with the provisos that

a) if

L denotes chlorine and

$R_1$ and $R_2$ together form a propylene group,

Q is other than hydrogen or S-benzyl,

b) if

Q represents formula B in which

$R_4$ and $R_5$ mean hydrogen atoms,

L stands for chlorine and

$R_2$ represents a methyl radical

$R_1$ is other than hydrogen and

c) if

Q represents formula A with

p = 0 and

$R_3$ = a benzyl radical,

L means chlorine and

$R_2$ stands for a methyl radical

$R_1$ is other than hydrogen

and their salts.

The invention encompasses all the tautomeric forms of the 1,2,4-triazolo[1,5-a]pyrimidine derivatives of the general formula I.

In the Prior Art for no compounds a gastric acid secretion inhibiting effect has been indicated and even those compounds for which an anti-ulcer activity has been indicated (Chemical Abstracts, Vol. 105, 1986, No. 97493k, page 640) are imidazopyrimidines structurally considerably different from the 1,2,4-triazolo[1,5-a]pyrimidine derivatives of the general formula I of the present invention. Furthermore compounds corresponding to such according to the invention in which, however, in the 5-position (that of L) is a hydroxy group have no antiulcer activity at all, whereas in Chemical Abstracts, Vol. 105, 1986, Abstract No. 97493k, page 640 a compound having in the 5-position a hydroxy group has been stressed as such having the greatest antiulcer activity.

The term "alkyl group" used throughout the specification relates to straight or branched chained saturated aliphatic

hydrocarbon groups containing the given number of carbon atom(s), e.g. methyl, ethyl, tert-butyl, n-butyl or n-octyl groups. The term "alkenyl group" designates straight or branched chained aliphatic hydrocarbon groups comprising at least one double bond, e.g. vinyl, allyl, prop-2-enyl, methylallyl or butenyl groups. The term "phenyl-($C_{1-4}$ alkyl)" relates to $C_{1-4}$ alkyl groups in which 1 or more, preferably 2, hydrogen atom(s) is/are replaced by [a] phenyl group(s), e.g. benzyl, 1-phenylethyl or 2-phenylethyl groups or di-(phenyl)-methyl, 1,1-di-(phenyl)-ethyl, 2,2-di-(phenyl)--ethyl or 1,2-di-(phenyl)-ethyl groups. The "alkylaminoalkyl" and "dialkylaminoalkyl" groups comprise alkyl groups containing the given number of carbon atom(s), e.g. methylaminomethyl, methylaminoethyl, ethylaminoisopropyl,dimethylaminopropyl, dimethylaminoethyl or diisopropylaminoethyl groups.

The term "halogen" encompasses the halogen atoms, that is fluorine, chlorine, bromine and iodine.

Preferably the halogen(s) which may be represented by L and/or which may be carried by the phenyl-($C_{1-4}$ alkyl) group which may be represented by $R_3$ is/are chlorine and/or bromine.

Furthermore it is preferred that the $C_{1-4}$ alkyl substituent which may be carried by the morpholino, piperidino or piperazinyl group which may be represented by Q and/or the alkyl part(s) of the phenyl-($C_{1-4}$ alkyl) group(s) which may be represented by $R_3$ and/or $R_4$ and/or $R_5$ and/or $R_6$ and/or the $C_{1-4}$ alkyl group(s) of the di-($C_{1-4}$ alkyl)-amino part(s) of the di-($C_{1-4}$ alkyl)-amino--($C_{1-6}$ alkyl) group(s) which may be represented by $R_4$ and/or $R_5$ and/or the $C_{1-4}$ alkyl group which may be represented by $R_1$ and/or $R_2$ is/are such one(s) having 1 or 2, particularly 1, carbon atom (s); and/or the $C_{1-8}$ alkyl group which may be represented by $R_3$ is such one having from 1 to 6, particularly from 1 to 3, carbon atom(s), most particularly a methyl, ethyl, propyl or 1-(methyl)-ethyl group, above all the first one; and/or the $C_{2-6}$ alkenyl group(s) which may be represented by $R_3$ or $R_4$ and/or $R_5$ is/are such one(s) having from 2 to 4, particularly 3, carbon atoms, most particularly [an] allyl group(s), and/or the $C_{1-12}$ alkyl group which may be represented by $R_4$ and/or $R_5$ is/are such one[s] having from 1 to 8, particularly 1 to 4, most particularly 1 or 2, carbon atom(s); or the $C_{1-6}$ alkyl group(s) of the amino-($C_{1-6}$ alkyl) part(s) of the di-($C_{1-4}$ alkyl)-amino-($C_{1-6}$ alkyl) group(s) which may be represented by $R_4$ and/or $R_5$ is/are such one(s) having from 1 to 4, particularly 1 to 3, most particularly 3, carbon atom(s).

Particularly preferred compounds according to the invention are 5-chloro-2-methylthio-6,7-dihydro-8H-cyclopenta [d]--1,2,4-triazolo[1,5-a]pyrimidine, 5-chloro-2-methylthio--6,7,8,9-tetrahydro-1,2,4-triazolo[5,1-b]quinazoline, 5-chloro-2-morpholino-6,7,8,9-tetrahydro-1,2,4-triazolo [5,1-b]quinazoline and 5-chloro-7-methyl-2-methylthio--1,2,4-triazolo[1,5-a]-pyrimidine.

As regards the nomenclature of the above compounds and in the entire further text it is referred to J. Heterocyclic Chem. 24 [1987], 1 503.

According to a further aspect of the present invention there is provided a process for the preparing the compounds according to the invention which is characterized in that a 1,2,4--triazolo[1,5-a]pyrimidine derivative of the general formula

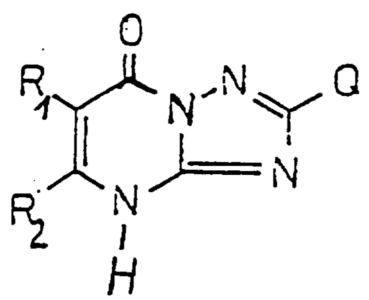

II,

wherein

Q, $R_1$ and $R_2$ are as stated above,

or the sodium salt thereof is reacted with a mineral acid halide of the general formula

$$A\text{-}X_i \qquad \text{III},$$

wherein

A denotes a mineral acid radical,
X stands for halogen and
i represents 1, 2, 3, 4 or 5

and, if desired, in a manner known per se converting a compound of the general formula I thus obtained into a salt or converting a salt of the compound of the general formula I thus obtained into the free base of formula I or into another salt.

In the process according to the invention for the reaction of a 1,2,4-triazolo[1,5-a]pyrimidine derivative of the general formula II or of the sodium salt thereofas mineral acid halide preferably phosphorus oxychloride, phosphorus pentachloride or thionyl chloride is used. The reaction can be performed without using any catalyst, but it is preferable to use a tertiary amine base, particularly pyridine, 1,1,3,3-tetramethylurea or N,N-dimethylaniline, or dimethylformamide, as catalyst. Furthermore it is preferred to use the latter in an amount of 0.1 to 120% by weight, particularly 10 to 40% by weight, related to the 1,2,4-triazolo[1,5-a]pyrimidine derivative of the general formula II. Advantageously the reaction is carried out in an inert solvent, particularly in benzene and/or in a homologue thereof, above all toluene and/or xylene, and/or in chloroform, dichloroethane and/or chlorobenzene, but it can also be performed without using any solvent. In the latter case suitably an excess of the mineral acid halide of the general formula III can be used as solvent. Suitably as temperature of the reaction from 20°C to the boiling point of the reaction mixture, preferably from 60°C to 120°C, are used. It is particularly preferable to carry out the reaction at a temperature from 80°C to 90°C.

The 1,2,4-triazolo[1,5-a]pyrimidine derivatives or the general formula I are organic bases, so that they can form salts with acids. As already mentioned, the salt formation can be carried out by methods known per se with organic or inorganic acids. The free base of the general formula I likewise can be liberated from a salt by conventional methods.

The 1,2,4-triazolo[1,5-a]pyrimidine derivatives of the general formula I thus obtained can be separated from the reaction mixture by methods known per se, generally by filtration, evaporation or extraction with a water-immiscible solvent, which operations are, in certain cases, preceded by the addition of some icy water to the reaction mixture.

The starting 1,2,4-triazolo[1,5-a]pyrimidine derivatives of the general formula II are known from the literature (J. Org. Chem. 25, 361 [1960]; J. Chem. Soc. 1961, 3 046; J. Org. Chem. 24, 779 [1959]; J. Heterocyclic Chem. 24, 1 503 [1987]; Org. Prep. Proced. Int. 21, 163 [1989]; Monatsh. Chem. 121, 173 [1990]; J. Heterocyclic Chem. 25, 1 497 [1988]; J. Heterocyclic Chem. 26, 971 [1989]).

The compounds of the general formula III, IV, V and VI are commercial products.

As already mentioned the 1,2,4-triazolo[1,5-a]pyrimidine derivatives of general formula I have useful pharmaceutical, particularly the gastric acid secretion inhibiting properties.

Hence by the invention also there are provided for medicaments which are characterized in that they contain as [an] active ingredient(s) at least one compound according to the invention, suitably in admixture with at least one inert solid and/or liquid pharmaceutical carrier and/or diluent and/or additive.

For proving the gastric acid secretion inhibiting effect tests were carried out according to the method of Shay (Shay, H., et al.: Gastroenterology 5, 45 [1945]). Rats of both sexes (Wistar breed, weighing 180-240 g) were fastened for 48 hours. On the day of the test the pylorus of the animals was bound under ether narcosis. The test compounds were administered orally 3 hours before the operation. 4 hours after the operation the animals were anaesthetized and the stomach was removed, the content thereof was centrifuged and the amount of the free acid was determined by titration, in the presence of Töpfer reagent. The inhibition in relation to the control is calculated in percentage. The results are summarized in the following Table.

Table

| Compound | | Dose mg/kg peroral | Inhibition % |
|---|---|---|---|
| Designation | No. of Example | | |
| 5-Chloro-2-methylthio-6,7-dihydro-8H-cyclopenta[d]-1,2,4-triazolo[1,5-a]pyrimidine | 2 | 30 | 73 |
| 5-Chloro-2-methylthio-6,7,8,9-tetrahydro-1,2,4-triazolo[5,1-b]quinazoline | 6 | 60 | 100 |
| 5-Chloro-2-morpholino-6,7,8,9-tetrahydro-1,2,4-triazolo[5,1-b]quinazoline | 12 | 200 | 85 |
| 5-Chloro-7-methyl-2-methylthio-1,2,4-triazolo[1,5-a]-pyrimidine | 49 | 50 | 67 |
| 3,4,5-Trimethoxythiobenzoic acid morpholide [Trithiozine] | Reference compound | 400 | 63 |

From the data of the above Table it can be established that the tested compounds according to the invention are

superior to the reference compound even at much lower doses.

Hence a further subject-matter of the invention is the use of the compounds according to the invention for preparing medicaments, particularly such having gastric acid secretion inhibiting effect.

Besides, the compounds according to the present invention can be used as intermediates for the preparation of 1,2,4-triazolo[1,5-a]pyrimidine derivatives of the general formula

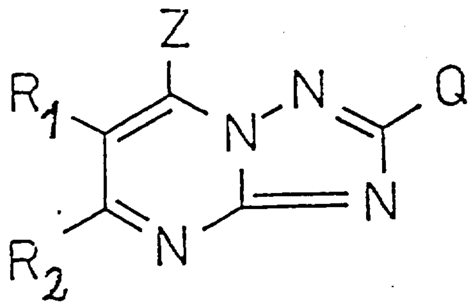

IV

wherein

Q, $R_1$ and $R_2$ are as above defined and

Z represents a group of the formula

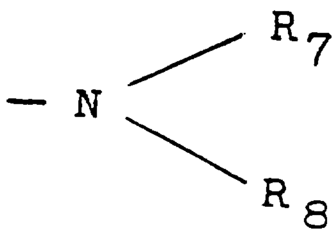

E

in which latter

$R_7$ and $R_8$ independently each represent hydrogen, straight or branched chained $C_{1-12}$ alkyl, optionally carrying one or more substituent(s) selected from the group consisting of hydroxy, carboxy, amino, morpholinocarbonyl, [4-(2'-{hydroxy}--ethyl)-piperazin-1-yl]--carbonyl and ($C_{1-4}$ alkoxy)-carbonyl or optionally carrying a saturated or unsaturated 4- to 8-membered, particularly 5- or 6-membered, heterocyclic group containing one or more nitrogen and/or oxygen atom(s) which latter may optionally be condensed with a benzene ring and/or optionally carry a $C_{1-4}$ alkyl or benzyl substituent; $C_{2-6}$ alkenyl, $C_{3-8}$ cycloalkyl, adamantyl or phenyl-($C_{1-4}$ alkyl), which latter may optionally carry one or more substituent(s) selected from the group consisting of halogen, hydroxy, $C_{1-4}$ alkyl, $C_{1-4}$ alkoxy, amino and nitro; ($C_{1-4}$ alkyl)--amino-($C_{1-8}$ alkyl) and di($C_{1-4}$ alkyl)--amino-($C_{1-8}$ alkyl); or

$R_7$ and $R_8$ together form a group of the formula

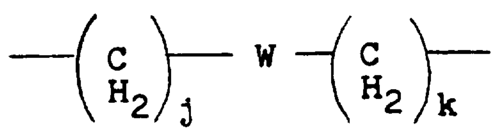

F

in which latter

j and k independently each represent 1, 2 or 3 and

W stands for oxygen or a

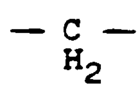

group or a

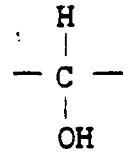

group or
a group of the formula

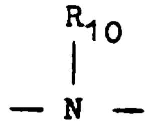

G

in which latter

$R_{10}$ denotes hydrogen, $C_{1-4}$ alkoxycarbonyl or $C_{1-4}$ alkyl, which latter may optionally carry one or more substituent(s) selected from the group consisting of hydroxy, ($C_{1-4}$ alkoxy)-carbonyl and phenyl; or

Z stands for a group of the formula

$$-S-R_9 \qquad H$$

wherein

$R_9$ represents $C_{1-4}$ alkyl, optionally substituted by a ($C_{1-4}$ alkoxy)-carbonyl group, with the proviso that
if Q represents a group of the formula A

$R_1$ and $R_2$ are other than hydrogen or $C_{1-4}$ alkyl,

and their salts. These compounds possess valuable pharmacological, particularly positive inotropic, antianginal, antiinflammatory and ulcus and gastric acid secretion inhibiting effects. They can be prepared by reacting the 1,2,4--triazolo [1,5-a]pyrimidine derivatives according to the invention with an amine or thiol of the general formula

$$H-Z \qquad V$$

wherein

Z is as stated above,

and optionally in a manner known per se converting a 1,2,4--triazolo[1,5-a]pyrimidine derivative of the formula VII thus obtained into a salt or converting a salt of the compound of the general formula VII thus obtained into the free base of formula VII or into another salt.

The definition of the groups is as indicated for those with regard to formula I, insofar they occur also there. Furthermore the term "alkoxy group" also as a part of a group relates to alkyl ether groups comprising $C_{1-4}$ alkyl groups. Examples for $C_{1-4}$ alkoxy groups are methoxy, ethoxy and tert-butoxy groups; examples for $C_{1-4}$ alkoxycarbonyl groups are methoxycarbonyl, ethoxycarbonyl and butoxycarbogroups. Moreover preferably the cycloalkyl group(s) which may be represented by $R_7$ and/or $R_8$ is/are such having 5 or 6 carbon atoms.

The invention is further illustrated by the following Examples.

Example 1

**5-Chloro-7-methyl-2-morpholino-1,2,4-triazolo[1,5-a]-pyrimidine**

To a suspension of 100.0 g (0.425 mole) of 7-methyl-2--morpholino-8H-1,2,4-triazolo[1,5-a]pyrimidin-5-one and 161.7 g (1.054 mole ≈ 98 ml) of phosphorus oxychloride in 80 ml of chloroform 33.3 g (0.42 mole ≈ 34 ml) of pyridine are added, and the reaction mixture is boiled for 1.5 hours under stirring. Then it is cooled and 500 ml of ice-cold water are added while cooling with icy water. The phases are separated, the aqueous phase is extracted with chloroform, the organic phases are combined and washed with ice-cold water, dried over anhydrous $Na_2SO_4$ and evaporated to dryness in vacuo. The crystalline product thus obtained is recrystallized from dimethylformamide.
Yield: 66.9 g (62 %)

M.p.: 175-176.5 °C.

Example 2

**5-Chloro-2-methylthio-6,7-dihydro-8H-cyclopenta[d]--1,2,4-triazolo[1,5-a]pyrimidine**

To a suspension of 100.0 g (0.45 mole) of 2-methylthio--6,7-dihydro-8H-cyclopenta[d]-1,2,4-triazolo[1,5-a]pyrimidin--5(9H)-one in 180 ml of toluene 170.0 g (1.109 mole ≈ 103 ml) of phosphorus oxychloride are added, then 37.2 g (0.47 mole ≈ 38 ml) of pyridine are introduced to the mixture. It is stirred at 60-65 °C for 3 hours, then the excess of phosphorus oxychloride and the toluene are distilled off in vacuo. Crushed ice is added to the oily residue, the separated crystals (93.3 g, 86.1 %) are filtered off, washed with ice-cold water and recrystallized from ethanol.
Yield: 78.5 g (72.5 %)
M.p.: 124-126 °C.

Example 3

**5-Chloro-2-methylthio-6,7-dihydro-8H-cyclopenta[d]--1,2,4-triazolo[1,5-a]pyrimidine**

To a suspension of 166.7 g (0.75 mole) of 2-methylthio-6,7-dihydro-8H-cyclopenta[d]-1,2,4-triazo1o[1,5-a]pyrimidin-5(9H)-one in 345.0 g (2.25 mole ≈ 206 ml) of phosphorus oxychloride 17.8 g (0.225 mole ≈ 18 ml) of pyridine are added and the reaction mixture is stirred at 60-65 °C for 5 hours. Then it is cooled and the crystalline mass thus obtained is admixed with 350 ml of n-hexane. The separated product is filtered, washed with hexane, then it is admixed with 1 kg of crushed ice, filtered and washed until neutral successively with ice-cold water, cold diluted $NaHCO_3$ solution and ice-cold water. The crude product (168.5 g, 93.3 %, m.p.: 119-121 °C) is recrystallized from isopropanol.
Yield: 149.9 g (83.0 %)
M.p.: 126-128 °C.

**Example 4**

**5-Chloro-2-morpholino-6,7-dihydro-8H-cyclopenta[d]--1,2,4-triazolo[1,5-a]pyrimidine**

To a suspension of 100.0 g (0.383 mole) of 2-morpholino-6,7-dihydro-8H-cyclopenta[d]-1,2,4-triazolo[1,5-a]-pyrimidin-5(9H)-one in 146.85 g (0.958 mole ≈ 89 ml) of phosphorus oxychloride 30.1 g (0.38 mole 30.7 ml) of pyridine are dropped, and the thick reaction mixture is stirred on an oil bath of 75 °C for 1.5 hours. The excess of phosphorus oxychloride is removed in vacuo, the oily product is rubbed with ice, then the separated product is filtered and washed with icy water. The crude product thus obtained (94.65 g, 88.45 %, m.p.: 213-215 °C) is recrystallized from dimethylformamide.
Yield: 56.4 g (52.7 %)
M.p.: 213-215 °C.

**Example 5**

**5-Chloro-2-morpholino-6,7-dihydro-8H-cyclopenta[d]--1,2,4-triazolo[1,5-a]pyrimidine**

To a suspension of 78 g (0.298 mole) of 2-morpholino--6,7-dihydro-8H-cyclopenta[d]-1,2,4-triazolo[1,5-a]pyrimidin--5(9H)-one in 153.3 g (1.00 mole, ≈ 93 ml) of phosphorus oxychloride 7.9 g (0.10 mole 8.0 ml) of pyridine are added, and the reaction mixture is stirred for 2.5 hours while the inner temperature is kept at 80 °C. The excess of phosphorus oxychloride is distilled off in vacuo, the brown oily residue is rubbed with 800 g of crushed ice and allowed to stand overnight at 5 °C. The separated product is filtered and washed successively with icy water, cold diluted $NaHCO_3$ solution and icy water until neutral. The crude product thus obtained (78.2 g, 93.8 %, m.p.: 212-217 °C) is recrystallized from isopropanol.
Yield: 60.95 g (73.0 %)
M.p.: 209-210 °C.

When extracting the aqueous mother liquor with chloroform further 2.1 g of crude product are obtained, which is recrystallized from 2-propanol.
Second crop: 1.6 g (1.9 %)
M.p.: 210-212 °C.

**Example 6**

**5-Chloro-2-methylthio-6,7,8,9-tetrahydro-1,2,4--triazolo[5,1-b]quinazoline**

To 132.0 g (0.86 mole ≈ 80 ml) of phosphorus oxychloride 55.5 g (0.215 mole) of 2-methylthio-6,7,8,9-tetrahydro-1,2,4-triazolo[5,1-b]quinazolin-5(10H)-one sodium salt are added within 30 minutes at such a rate that the temperature of the reaction mixture should not exceed 80 °C. Then the suspension is cooled to 65 °C, 6.8 g (0.086 mole ≈ 7.0 ml) of pyridine are dropped to it and the reaction mixture is stirred for 10 hours while the inner temperature is kept at 80 °C. The mixture is allowed to stand overnight, the separated crystals are filtered and washed successively with ether, icy water, cold diluted $NaHCO_3$ solution and water.
Yield: 40.56 g (74.0 %)
M.p.: 123-125 °C.

When recrystallizing a small sample from isopropanol, the melting point of the product raises to 126-128 °C.

**Example 7**

**5-Chloro-2-methylthio-6,7,8,9-tetrahydro-1,2,4-triazolo[5,1-b]quinazoline dichlorophosphate**

To a suspension of 201.6 g (0.85 mole) of 2-methylthio--6,7,8,9-tetrahydro-1,2,4-triazolo[5,1-b]quinazolin-5(10H)--one in 510 ml (841.5 g ≈ 5.49 mole) of phosphorus oxychloride 19.75 g (0.25 mole ≈ 20 ml) of pyridine are added under stirring, and the reaction mixture is stirred at 105-110 °C for 2 hours. The solution thus obtained is cooled and seeded, the separated crystals are filtered and washed with acetonitrile.
Yield: 211.5 g (63.6 %)
M.p.: 131-137 °C (decomp.) ,

**Example 8**

**2-Ethylthio-5-chloro-6,7,8,9-tetrahydro-1,2,4-triazolo-[5,1-b]quinazoline**

To a suspension of 15.02 g (0.06 mole) of 2-ethylthio--6,7,8,9-tetrahydro[5,1-b]quinazolin-5(10H)-one in 55.3 g (0.36 mole ≈ 33.5 ml) of phosphorus oxychloride 0.95 g (0.012 mole ≈ 0.97 ml) of pyridine are added, and the reaction mixture is stirred at 90-95 °C for 1.5 hours. The yellow solution thus obtained is cooled and added to 100 ml of n-heptane under stirring. The crystals thus obtained are filtered and washed until neutral successively with n-heptane, icy water, cold diluted $NaHCO_3$ solution and icy water.
Yield: 12.72 g (78.8 %)
M.p.: 118-120 °C.

When recrystallizing a small sample from ethyl acetate the melting point of the product raises to 121.5-123 °C.

Example 9

2-[1'-(Methyl)-ethyl-thio]-5-chloro-6,7,8,9-tetrahydro--1,2,4-triazolo[5,1-b]quinazoline

To a suspension of 15.86 g (0.06 mole) of 2-[1'-(methyl)--ethyl-thio]-6,7,8,9-tetrahydro[5,1-b]quinazolin-5(10H)-one in 46.2 g (0.30 mole ≈ 28.0 ml) of phosphorus oxychloride 0.95 g (0.012 mole 0.97 ml) of pyridine is added, and the reaction mixture is stirred at 90-95 °C for 1.5 hours. Then ice is added to it, the mixture is extracted with chloroform, the organic phase is extracted successively with water, cold diluted $NaHCO_3$ solution and icy water, dried over anhydrous $Na_2SO_4$ and evaporated to dryness in vacuo. Thus 16.4 g of red oil are obtained, which is purified by chromatography on a silica gel column and recrystallized from ethyl acetate.
Yield: 10.05 g (59.2 %)
M.p.: 90.5-91.5 °C .

**Example 10**

**5-Chloro-2-methylsulfonyl-6,7,8,9-tetrahydro-1,2,4--triazolo[5,1-b]quinazoline**

To a suspension of 2.68 g (0.01 mole) of 2-methylsulfonyl-6,7,8,9-tetrahydro-1,2,4-triazolo[5,1-b]quinazolin--5 (10H)-one in 16.5 g (0.108 mole ≈ 10.0 ml) of phosphorus oxychloride 10 drops of pyridine are added, and the reaction mixture is stirred on an oil bath of 100 °C for 12 hours. The product is precipitated from the solution thus obtained with

25 ml of diethyl ether, the ether-phosphorus oxychloride supernatant layer is decanted, to the residue 25 g of crushed ice are added, the separated crystals are filtered and washed successively with icy water, cold diluted $NaHCO_3$ solution, icy water and isopropanol. The crude product thus obtained (2.23 g) is purified by column chromatography (Kieselgel 60H).
Yield: 1.18 g (41.1 %)
M.p.: 199-201 °C .

**Example 11**

**5-Bromo-2-methylthio-6,7,8,9-tetrahydro-1,2,4-triazolo-[5,1-b]quinazoline**

12.76 g (0.054 mole) of 2-methylthio-6,7,8,9-tetrahydro-1,2,4-triazolo[5,1-b]quinazolin-5(10H)-one are admixed with 31.0 g (0.108 mole) of freshly distilled phosphorus oxybromide, and the reaction mixture is melted on an oil bath of about 60 °C. When the mixture has become miscible 0.39 g (0.005 mole ≈ 0.40 ml) of pyridine is added and the reaction mixture is stirred at 90-95 °C for 3 hours. Then it is cooled to 60 °C, 100 ml of chloroform are added and the mixture is treated with 250 g of crushed ice. The phases are separated, the aqueous phase is extracted with chloroform, the organic phases are combined, washed with icy water, dried over anhydrous $Na_2SO_4$ and evaporated to dryness in vacuo. Thus 9.8 g of brown oil are obtained which is purified by column chromatography (Kieselgel 60H) and recrystallized from acetonitrile.
Yield: 3.15 g (19.6 %)
M.p.: 131.5-133.5 °C.

**Example 12**

**5-Chloro-2-morpholino-6,7,8,9-tetrahydro-1,2,4--triazolo[5,1-b]quinazoline**

To a suspension of 27.53 g (0.10 mole) of 2-morpholino--6,7,8,9-tetrahydro-1,2,4-triazolo[5,1-b]quinazolin-5 (10H)--one in 76.7 g (0.50 mole ≈ 46.5 ml) of phosphorus oxychloride 1.57 g (0.02 mole ≈ 1.6 ml) of pyridine are added and the reaction mixture is stirred for 2 hours while keeping the inner temperature at 90-95 °C. The yellow solution is cooled and poured onto 100 ml of n-heptane under stirring. The separated crystals are filtered and washed successively with n-heptane, icy water, cold diluted $NaHCO_3$ solution, icy water and 2-propanol.
Yield: 21.2 g (72.2 %)
M.p.: 169-171 °C .

When recrystallizing a small sample from isopropanol, the melting point raises to 170-171 °C.

**Example 13**

**5-Chloro-2-methylthio-6,7,8,9-tetrahydro-10H-cyclo-hepta[d]1,2,4-triazolo[1,5-a]pyrimidine**

To a suspension of 15.02 g (0.06 mole) of 2-methylthio--6,7,8,9-tetrahydro-loft-cyclohepta[d]-1,2,4-triazolo[1,5-a] pyrimidin-5(11H)-one in 49.5 g (0.323 mole ≈ 30 ml) of phosphorus oxychloride 0.49 g (0.0062 mole ≈ 0.50 ml) of pyridine is added, and the reaction mixture is stirred for 2 hours while the inner temperature is kept at 90-92 °C. The yellow solution thus obtained is cooled and then diluted with 50 ml of diethyl ether. The separated crystals are filtered and washed successively with ether, icy water, cold diluted $NaHCO_3$ solution, icy water and 2-propanol.
Yield: 14.78 g (91.6 %)
M.p.: 119.5-120.5 °C .

When extracting the aqueous-2-propanolic mother liquor with chloroform, 0.68 g (4.2 %) of a second crop is obtained which is recrystallized from ethyl acetate.
M.p.: 119-120.5 °C.

**Example 14**

**5-Chloro-2-morpholino-6,7,8,9-tetrahydro-10H-cyclohepta[d]-1,2,4-triazolo[1,5-a]pyrimidine**

To a suspension of 1.45 g (0.005 mole) of 2-morpholino--6,7,8,9-tetrahydro-10H-cyclohepta[d]-1,2,4-triazolo[1,5-a] pyrimidin-5(11H)-one in 4.95 g (0.032 mole ≈ 3 ml) of phosphorus oxychloride 5 drops of pyridine are added, and the reaction mixture is stirred for 1 hour while the inner temperature is kept at 70-72 °C. The solution thus obtained is then cooled, diluted with 15 ml of diethyl ether, and the solvent is decanted off the separated yellow oil. The residual oily

product is rubbed with 10.0 g of crushed ice, filtered and washed successively with icy water, cold diluted $NaHCO_3$ solution, icy water and cold isopropanol.
Yield: 1.00 g (64.9 %)
M.p.: 154-157 °C.
When recrystallizing a small sample from ethyl acetate, the melting point raises to 154-156 °C.

**Example 15**

**5-Chloro-2-methylthio-6,7,8,9,10,11-hexahydro-cycloocta[d]-1,2,4-triazolo[1,5-a]pyrimidine**

To a suspension of 0.71 g (2.7 mmoles) of 2-methylthio--6,7,8,9,10,11-hexahydro-cycloocta[d]-1,2,4-triazolo [1,5-a]-pyrimidin-5(12H)-one in 4.95 g (0.032 mole ≈ 3.0 ml) of phosphorus oxychloride 5 drops of pyridine are added, and the reaction mixture is stirred on an oil bath of 85 °C for 2 hours. Then it is cooled and a mixture of 50 g of crushed ice and 20 ml of dichloromethane is added to the yellow solution. The phases are separated, the aqueous phase is washed with dichloromethane, the organic phases are combined, washed successively with icy water, cold diluted $NaHCO_3$ solution and icy water, dried over $Na_2SO_4$ and evaporated to dryness in vacuo.
Yield: 0.68 g (89 %)
M.p.: 89-92 °C.
When recrystallizing a small sample from cyclohexane, the melting point raises to 90.5-92 °C.

**Example 16**

**5-Chloro-2-morpholino-6,7,8,9,10,11-hexahydro-cycleocta[d]-1,2,4-triazolo[1,5-a]pyrimidine**

To a suspension of 1.00 g (3.3 mmoles) of 2-morpholino--6,7,8,9,10,11-hexahydro-cycloocta[d]-1,2,4-triazolo [1,5-a]-pyrimidin-5(12H)-one in 4.95 g (0.032 mole ≈ 3.0 ml) of phosphorus oxychloride 5 drops of pyridine are added, and the reaction mixture is stirred on an oil bath of 85 °C for 1 hour. Further processing of the mixture is carried out according to Example 15.
Yield: 0.98 g (92.5 %)
M.p.: 142-145 °C.
When recrystallizing a small sample from ethyl acetate, the melting point raises to 143-145 °C.

**Example 17**

**5-Chloro-2-methylthio-6,7,8,9,10,11,12,13,14,15-decahydro-cyclododeca[d]-1,2,4-triazolo[1,5-a]pyrimidine**

To a suspension of 3.52 g (0.011 mole) of 2-methylthio--6,7,8,9,10,11,12,13,14,15-decahydro-cyclododeca[d]-1,2,4--triazolo[1,5-a]pyrimidin-5(16H)-one in 16.5 g (0.108 mole, 10 ml) of phosphorus oxychloride 0.39 g (0.005 mole, ≈ 0.40 ml) of pyridine is added, and the reaction mixture is stirred on an oil bath of 85 °C for 6 hours. Then it is cooled and a mixture of 100 g of crushed ice and 30 ml of chloroform is added to the orange solution. The phases are separated, the aqueous phase is washed with chloroform, the organic phases are combined and washed successively with icy water, cold diluted $NaHCO_3$ solution and icy water, dried over anhydrous $Na_2SO_4$ and evaporated to dryness in vacuo.
Yield: 3.40 g (91 %)
M.p.: 126.5-128.5 °C .
When recrystallizing a small sample from ethyl acetate, the melting point raises to 128-129 °C.

**Example 18**

**5-Chloro-2-morpholino-6,7,8,9,10,11,12,13,14,15-decahydro-cyclododeca[d]-1,2,4-triazolo[1,5-a]pyrimidine**

To a suspension of 1.08 g (0.003 mole) of 2-morpholino--6,7,8,9,10,11,12,13,14,15-decahydro-cyclododeca[d]-1,2,4--triazolo[1,5-a]pyrimidin-5(16H)-one in 4.95 g (0.032 mole, ≈ 3.0 ml) of phosphorus oxychloride 5 drops of pyridine are added, and the reaction mixture is stirred on an oil bath of 85 °C for 4 hours. Then it is cooled and a mixture of 40 g of crushed ice and 20 ml of dichloromethane is added to the yellow solution. The phases are separated, the aqueous phase is washed with dichloromethane, the organic phases are combined, washed successively with icy water, cold diluted $NaHCO_3$ solution and icy water, dried over $Na_2SO_4$ and evaporated to dryness in vacuo.
Yield: 1.05 g (92.6 %)
M.p.: 157-163 °C.

When recrystallizing a small sample from ethyl acetate, the melting point raises to 161.5-163 °C.

**Example 19**

**5-Chloro-2-methylthio-7,8-dihydro-9H-thiopyrano[3,2-d]1,2,4-triazolo[1,5-a]pyrimidine**

To a suspension of 6.87 g (0.027 mole) of 2-methylthio--7,8-dihydro-9H-thiopyrano[3,2-d]-1,2,4-triazolo[1,5-a]pyrimidin-5(10H)-one in 44.55 g (0.29 mole ≈ 27.0 ml) of phosphorus oxychloride 0.235 g (0.003 mole ≈ 0.24 ml) of pyridine is added, and the reaction mixture is stirred at 90 °C for 12 hours. Then further 4.95 g (0.0323 mole ≈ 3.0 ml) of phosphorus oxychloride and a few drops of pyridine are added to the yellow suspension thus obtained, and the mixture is stirred for further 5 hours at 90 °C. To complete the reaction 16.5 g (0.108 mole ≈ 10.0 ml) of phosphorus oxychloride and 0.49 g (0.062 mole ≈ 0.50 ml) of pyridine are added to the reaction mixture and it is stirred for further 15 hours at 90 °C. To the thus-obtained brown solution, from which a few crystals get separated upon cooling, 80.0 ml of diethyl ether are added under stirring. The solvent is decanted off the separated brown oil. Then the addition of diethyl ether and the decantation are repeated. The oil is rubbed with 150 g of crushed ice, whereupon it disintegrates into brown crystals. The crystals are filtered and washed successively with icy water, cold diluted $NaHCO_3$ solution and icy water.
Yield: 6.84 g (92.9 %)
M.p.: 140-142 °C .

When purifying a small sample by chromatography on a Kieselgel 60H column and recrystallizing from ethyl acetate, the melting point raises to 142-143 °C.

**Example 20**

**2-Dimethylamino-5-chloro-7,8-dihydro-9H-thiopyrano[3,2-d]-1,2,4-triazolo[1,5-a]pyrimidine**

To a suspension of 22.62 g (0.09 mole) of 2-dimethylamino-7,8-dihydro-9H-thiopyrano[3,2-d]-1,2,4-triazolo[1,5-a]-pyrimidin-5(10H)-one in 82.8 g (0.54 mole ≈ 50.2 ml) of phosphorus oxychloride 1.42 g (0.018 mole ≈ 1.45 ml) of pyridine are added, and the reaction mixture is stirred for 4 hours while keeping the inner temperature at 90 °C. The mixture is then cooled, the separated crystals are filtered and washed successively with phosphorus oxychloride, diisopropyl ether, icy water, cold diluted $NaHCO_3$ solution and icy water.
Yield: 17.3 g (71.2 %)
M.p.: 142-146 °C.

When purifying a small sample by column chromatography on a Kieselgel 60H column and recrystallizing from ethyl acetate, the melting point raises to 143-145 °C.

**Example 21**

**8-Benzyl-5-chloro-2-methylthio-6,7,8,9-tetrahydropyrido[3,4-d]-1,2,4-triazolo[1,5-a]pyrimidine**

13.98 g (0.04 mole) of 1-benzyl-2-methylthio-6,7,8,9--tetrahydropyrido[3,4-d]-1,2,4-triazolo[1,5-a]pyrimidin--5(10H)-one sodium salt are added to 42.93 g (0.28 mole ≈ 26.0 ml) of phosphorus oxychloride in small portions, and 0.63 g (0.008 mole ≈ 0.64 ml) of pyridine is dropped to the suspension thus obtained at 45 °C. The reaction mixture is stirred on an oil bath of 80 °C for 1 hour, then it is cooled, the separated crystals are filtered and washed successively with phosphorus oxychloride, ether and acetonitrile to yield the first crop of the hydrochloride of the aimed product. The mother liquor is diluted with ether, the supernatant layer is decanted off the separated oil, the residue is dissolved in chloroform, treated with 150 g of crushed ice and the phases are separated. The aqueous phase is washed with chloroform, the organic phases are combined, washed with icy water and cold diluted $NaHCO_3$ solution and dried over anhydrous $Na_2SO_4$. The first crop of hydrochloride is dissolved in the chloroform solution obtained as specified above, 5.8 g (0.057 mole 8.0 ml) of triethylamine are added and the mixture is allowed to stand for a few minutes. The triethylamine hydrochloride formed in the reaction is removed by washing with water, the organic phase is dried over anhydrous $Na_2SO_4$ and evaporated in vacuo. The residual 9.1 g of brown oil is triturated with 20 ml of ethyl acetate to obtain a crystalline product. The separated crystals are filtered and washed with cold ethyl acetate.
Yield: 7.6 g (55.0 %)
M.p.: 90-99 °C .

When purifying a small sample by chromatography on a Kieselgel 60H column followed by suspending it in diethyl ether, the melting point raises to 98-101 °C.

**Example 22**

**a) 7-Benzyl-5-chloro-2-methylthio-6,7,8,9-tetrahydropyrido[4,3-d]-1,2,4-triazolo[1,5-a]pyrimidine hydrochloride**

To a suspension of 10.64 g (0.032 mole) of 7-benzyl-2--methylthio-6,7,8,9-tetrahydropyrido[4,3-d]-1,2,4-triazolo [1,5-a]pyrimidin-5(10H)-one in 41.25 g (0.269 mole ≈ 25.0 ml) of phosphorus oxychloride 0.59 g (0.0074 mole ≈ 0.6 ml) of pyridine is dropped, and the reaction mixture is stirred on an oil bath of 95 °C for 5 hours. The brown suspension is then cooled, filtered and washed successively with phosphorus oxychloride, ether and acetone.
Yield: 8.60 g (69.2 %)
M.p.: 205-220 °C (decomp.).

**b) 7-Benzyl-5-chloro-2-methylthio-6,7,8,9-tetrahydropyrido[4,3-d]-1,2,4-triazolo[1,5-a]pyrimidine**

8.6 g of the hydrochloride salt obtained according to variant a) are dissolved in 30.0 ml of chloroform containing 2.32 g (0.023 mole ≈ 3.2 ml) of triethylamine. The thus- formed triethylamine hydrochloride is extracted with water, the chloroform solution is dried over anhydrous $Na_2SO_4$, the brown solution is passed through a Kieselgel 60H layer of about 0.5 cm and evaporated to dryness in vacuo. To the residueal oily product a few drops of ether are added, the separated crystals are filtered off, washed with ether and recrystallized from acetonitrile.
Total yield: 6.14 g (54.6 %)
M.p.: 163-165 °C.

**Example 23**

**2-Diallylamino-5-chloro-6,7-dihydro-8H-cyclopenta[d]--1,2,4-triazolo[1,5-a]pyrimidine**

To a suspension of 5.61 g (0.0207 mole) of 2-diallylamino-6,7-dihydro-8H-cyclopenta[d]-1,2,4-triazolo[1,5-a]pyrimidin-5(9H)-one in 16.5 g (0.108 mole ≈ 10 ml) of phosphorus oxychloride 0.395 g (0.005 mole ≈ 0.40 ml) of pyridine are added, and the reaction mixture is stirred on an oil bath of 80 °C for half an hour. The yellow solution is then cooled and poured onto 150 g of crushed ice. When the ice has melted, the separated crystals are filtered and washed successively with icy water, cold diluted $NaHCO_3$ solution and icy water.
Yield: 3.85 g (64.2 %)
M.p.: 57-59 °C.

When recrystallizing a small sample from n-hexane, the melting point of the product raises to 59-60.5 °C.

**Example 24**

**2-Diallylamino-5-chloro-6,7-dihydro-8H-cyclopenta[d]--1,2,4-triazolo[1,5-a]pyrimidine**

To a suspension of 8.14 g (0.03 mole) of 2-diallylamino-6,7-dihydro-8H-cyclopenta[d]-1,2,4-triazolo[1,5-a]-pyrimidin-5(9H)-one in 16.5 g (0.108 mole ≈ 10 ml) of phosphorus oxychloride 0.395 g (0.005 mole ≈ 0.40 ml) of pyridine are added, and the reaction mixture is stirred on an oil bath of 80 °C for 0.5 hour. The yellow solution is cooled and poured onto 150 g of crushed ice, then 100 ml of chloroform are added to it. When the ice has already melted, the phases are separated, the aqueous phase is extracted with chloroform, the organic phases are combined and washed successively with icy water, cold diluted $NaHCO_3$ solution and icy water, dried over anhydrous $Na_2SO_4$ and evaporated to dryness in vacuo. Thus 7.7 g of oily product are obtained, which is purified by chromatography on a silica gel column (eluent: a 10:1 (by volume) mixture of n-hexane and benzene) and recrystallized from n-hexane.
Yield: 5.73 g (65.9 %)
M.p.: 59-61°C .

**Example 25**

**2-Diallylamino-5-chloro-6,7-dihydro-8H-cyclopenta[d]--1,2,4-triazolo[1,5-a]pyrimidine**

To a suspension of 0.54 g (0.002 mole) of 2-diallylamino-6,7-dihydro-8H-cyclopenta[d]-1,2,4-triazolo[1,5-a]-pyrimidin-5(9H)-one in 1.65 g (0.0323 mole ≈ 1.00 ml) of phosphorus oxychloride 2 drops of pyridine are added, and the reaction mixture is stirred at 25 °C for 5 hours. Then the brown solution is poured onto 15 g of crushed ice and 10 ml of chloroform are added to it. When the ice has already melted, the phases are separated, the aqueous phase is

extracted with chloroform, the organic phases are combined, washed successively with icy water, cold diluted $NaHCO_3$ solution and icy water, dried over anhydrous $Na_2SO_4$ and evaporated to dryness in vacuo.

Thus 0.44 g (75.9 %) of crude product is obtained, which is dissolved in a 10:1 (by volume) mixture of n-hexane and benzene, purified by chromatography on a short silica gel column, evaporated again and recrystallized from n-hexane.
Yield: 0.30 g (51.4 %)
M.p.: 59-60.5 % .

**Example 26**

**2-Diallylamino-5-chloro-6,7-dihydro-8H-cyclopenta[d]--1,2,4-triazolo[1,5-a]pyrimidine**

One proceeds according to Example 25 except that instead of pyridine 2 drops of 1,1,3,3-tetramethylurea are used as catalyst, the reaction is carried out at 70 °C instead of 25 °C, and the reaction mixture is stirred for 15 minutes instead of for 5 hours. Thus 0.40 g (69 %) of crude product is obtained, which is dissolved in a 10:1 (by volume) mixture of n-hexane and benzene, purified by chromatography on a short silica gel column, evaporated again and recrystallized from n-hexane.
Yield: 0.32 g (55.2 %)
M.p.: 58-60 °C.

**Example 27**

**2-Diallylamino-5-chloro-6,7-dihydro-8H-cyclopenta[d]--1,2,4-triazolo[1,5-a]pyrimidine**

One proceeds according to Example 25 except that instead of pyridine 2 drops of N,N-dimethylformamide are used as catalyst, the reaction is carried out at 70 °C instead of at 25 °C, and the reaction mixture is stirred for 15 minutes instead of for 5 hours. Thus 0.37 g (63.8 %) of crude product is obtained, which is dissolved in a 10:1 (by volume) mixture of n-hexane and benzene, purified by chromatography on a short silica gel column, evaporated again and recrystallized from n-hexane.
Yield: 0.28 g (48.2 %)
M.p.: 56-60 °C.

**Example 28**

**2-Diallylamino-5-chloro-6,7-dihydro-8H-cyclopenta[d]--1,2,4-triazolo[1,5-a]pyrimidine**

One proceeds according to Example 25 except that instead of pyridine 2 drops of N,N-dimethylaniline are used as catalyst, the reaction is carried out at 70 °C instead of at 25 °C, and the reaction mixture is stirred for 15 minutes instead of for 5 hours. Thus 0.42 g (72.5 %) of crude product are obtained, which is dissolved in a 10:1 mixture of n-hexane and benzene (by volume), purified by chromatography using a short silica gel column, evaporated again and recrystallized from n-hexane.
Yield: 0.36 g (62.0 %)
M.p.: 58-59.5 °C .

**Example 29**

**2-Diallylamino-5-chloro-6,7-dihydro-8H-cyclopenta[d]--1,2,4-triazolo[1,5-a]pyrimidine**

One proceeds according to Example 25 except that no catalyst is used, the reaction is carried out at 70 °C instead of at 25 °C, and the reaction mixture is stirred for half an hour instead of for 5 hours. Thus 0.39 g (67.2 %) of crude product is obtained which is dissolved in a 10:1 (by volume) mixture of n-hexane and benzene, purified by chromatography using a short silica gel column, evaporated again and recrystallized from n-hexane.
Yield: 0.15 g (25.9 %)
M.p.: 58-60 °C .

Example 30

5-Chloro-2-methylsulphinyl-6,7,8,9-tetrahydro-1,2,4--triazolo[5,1-b]quinazoline

To a suspension of 4.77 g (0.019 mole) of 2-methylsulphinyl-6,7,8,9-tetrahydro-1,2,4-triazolo[5,1-b]quinazolin--5(10H)-one in 33.0 g (0.216 mole ≈ 20 ml) of phosphorus oxychloride 20 drops of pyridine are added and the reaction mixture is stirred on an oil bath of 100 °C for 12 hours. The product is precipitated from the obtained solution with 50 ml of diethyl ether. The ether-phosphorus oxychloride supernatant layer is decanted, to the residue 50 g of crushed ice are added, the separated crystals are filtered and washed successively with icy water, diluted $NaHCO_3$ solution, icy water and isopropanol. The crude product thus obtained is purified by column chromatography (Kieselgel 60H).
Yield: 3.67 g (71.4 %)
M.p.: 136-138 °C (after recrystallization from ethyl acetate).

**Example 31**

**5-Chloro-2-morpholino-8,9-dihydro-6H-thiopyrano[4,3-d]-1,2,4-triazolo[1,5-a]pyrimidine**

To a suspension of 1.47 g (0.005 mole) of 2-morpholino--8,9-dihydro-thiopyrano[4,3-d]-1,2,4-triazolo[1,5-a]-pyrimidin-5(10H)-one in 7.43 g (0.048 mole ≈ 4.5 ml) of phosphorus oxychloride 8 drops of pyridine are added, and the reaction mixture is stirred on an oil bath of 85 °C for 1.5 hours. Then it is worked up according to Example 18.
Yield: 1.1 g (70.5 %)
M.p.: 171-174 °C (after recrystallization from acetonitrile).

**Example 32**

**5-chloro-2-(n-octylamino)-6,7-dihydro-8H-cyclopenta[d]-1,2,4-triazolo[1,5-a]pyrimidine**

To a suspension of 7.59 g (0.025 mole) of 2-(n-octylamino)-6,7-dihydro-8H-cyclopenta[d]-1,2,4-triazolo[1,5-a]pyrimidin-5(9H)-one in 4.13 g (0.027 mole ≈ 2.5 ml) of phosphorus oxychloride 3 drops of pyridine are added and the reaction mixture is stirred at a temperature of 80 °C for 2.5 hours, then it is worked up as described in Example 10.
Yield: 4.08 g (50.7 %)
M.p.: 142-145 °C.

**Example 33**

**2-(n-Hexylthio)-5-chloro-6,7,8,9-tetrahydro-1,2,4-triazolo[5,1-b]quinazoline**

To a suspension of 2.14 g (0.007 mole) of 2-(n-hexylthio)-6,7,8,9-tetrahydro-1,2,4-triazolo[5,1-b]quinazolin--5(10H)-one in 4.6 g (0.03 mole ≈ 2.8 ml) of phosphorus oxychloride 0.095 g (0.0012 mole ≈ 0.1 ml) of pyridine is added, and the reaction mixture is stirred at 70 °C for 8 hours, then it is worked up as described in Example 9.
Yield: 1.50 g (66.1 %) of oily product (it gets crystalline upon standing in a refrigerator, m.p.: ≈ 18 °C).

**Example 34**

**2-Allylthio-5-chloro-6,7,8,9-tetrahydro-1,2,4-triazolo-[5,1-b]quinazoline**

To a suspension of 2.47 g (0.0094 mole) of 2-allylthio--6,7,8,9-tetrahydro-1,2,4-triazolo[5,1-b]quinazolin--5(10H)-one in 16.5 g (0.108 mole ≈ 10.0 ml) of phosphorus oxychloride 0.74 g (0.0094 mole ≈ 0.76 ml) of pyridine is added, and the reaction mixture is warmed on an oil bath of 85 °C for 1 hour. Then 100 g of crushed ice are added to the solution thus obtained and the separated crystals are washed successively with water, diluted $NaHCO_3$ solution, water and a slight amount of isopropanol.
Yield: 1.72 g (65 %)
M.p.: 76.5-77.5 °C (after recrystallization from a mixture of n-hexane and diisopropyl ether in a ratio by volume/volume of 10 : 17.

**Example 35**

**2-Benzylthio-5-chloro-6,7,8,9-tetrahydro-1,2,4-triazolo[5,1-b]quinazoline**

To a suspension of 2.19 g (0.007 mole) of 2-benzylthio--6,7,8,9-tetrahydro-1,2,4-triazolo[5,1-b]quinazolin-5(10H)--one in 8.25 g (0.0538 mole ≈ 5.0 ml) of phosphorus oxychloride 5 drops of pyridine are added and the mixture is warmed on an oil bath of 85 °C for 11 hours. Then 50 g of crushed ice are added to the solution thus obtained, the separated oily product is extracted with dichloromethane, the dichloromethane solution is extracted with water, diluted $NaHCO_3$ solution and water again, dried and evaporated to dryness. The crude product thus obtained is purified by column chromatography (Kieselgel 60H).
Yield: 1.80 g (77.6 %)
M.p.: 145-147 °C (after recrystallization from ethyl acetate).

**Example 36**

**5-Chloro-2-[4'-(nitro)-benzyl-thio]-6,7,8,9-tetrahydro--1,2,4-triazolo[5,1-b]quinazoline**

To a suspension of 2.14 g (0.006 mole) of 2-[4'-(nitro)--benzyl-thio]-6,7,8,9-tetrahydro-1,2,4-triazolo[5,1-b]quinazolin-5(10H)-one in 8.25 g (0.0538 mole ≈ 5.0 ml) of phosphorus oxychloride 5 drops of pyridine are added, and the mixture is stirred on an oil bath of 80 °C for 20 hours. The yellow solution thus obtained is treated with 50 g of crushed ice. The separated crystals are filtered and washed successively with water, diluted $NaHCO_3$ solution, water and diethyl ether.
Yield: 2.11 g (93.8 %)
M.p.: 174-179 °C .

When purifying a small sample on a Kieselgel 60H column, the melting point raises to 183-185 °C.

**Example 37**

**5-Chloro-2-[4'-(chloro)-benzyl-thio]-6,7,8,9-tetrahydro--1,2,4-triazolo[5,1-b]quinazoline**

One proceeds according to Example 36 except that 2.08 g (0.006 mole) of 2-[4'-(chloro)-benzyl-thio]-6,7,8,9-tetrahydro--1,2,4-triazo1o[5,1-b]quinazolin-5(10H)-one are used as starting substance.
Yield: 1.94 g (88.6 %)
M.p.: 160-161 °C (after recrystallization from ethyl acetate).

**Example 38**

**5-Chloro-2-[2'-(nitro)-4'-(trifluoromethyl)-phenyl-thio]--6,7,8,9-tetrahydro-1,2,4-triazolo[5,1-b]quinazoline**

One proceeds according to Example 36 except that 2.06 g (0.005 mole) of 2-[2'-(nitro)-4'-(trifluoromethyl)-phenyl-thio]--6,7,8,9-tetrahydro-1,2,4-triazolo[5,1-b]quinazolin-5(10H)--one are used as starting substance.
Yield: 2.08 g (96.7 %)
M.p.: 150-158 °C.

When purifying a small sample on a Kieselgel 60H column and recrystallizing it from acetonitrile, the melting point raises to 179.5-180.5 °C.

**Example 39**

**2-Ethylamino-5-chloro-6,7-dihydro-8H-cyclopenta[d]-1,2,4-triazolo[1,5-a]pyrimidine**

One proceeds according to Example 35 except that 6.57 g (0.03 mole) of 2-ethylamino-6,7-dihydro-8H-cyclopenta[d]--1,2,4-triazalo[1,5-a]pyrimidin-5(9H)-one are used as starting substance and also the other reactants are applied in proportionally reduced amounts.
Yield: 4.47 g (62.7 %)
M.p.: 212-214 °C (decomp.) after recrystallization from a mixture of ethyl acetate and cyclohexane in a ratio by volume/volume of 1 : 1.

**Example 40**

**2-Benzylamino-5-chloro-6,7,8,9-tetrahydro-1,2,4--triazolo[5,1-b]quinazoline**

One proceeds according to Example 39 except that 7.38 g (0.025 mole) of 2-benzylamino-6,7,8,9-tetrahydro-1,2,4--triazolo[5,1-b]quinazolin-5(10H)-one are used as starting substance and also the other reactants are applied in proportionally reduced amounts.
Yield: 5.54 g (70.7 %)
M.p.: 177-179 °C (after purification by chromatography on a Kieselgel 60H column and recrystallization from acetonitrile) .

**Example 41**

**5-Chloro-2-[4'-(methyl)-piperazin-1'-yl]-6,7-dihydro-8H--cyclopenta[d]-1,2,4-triazolo[1,5-a]pyrimidine**

To a suspension of 20.57 g (0.075 mole) of 2-[4'--(methyl)-piperazin-1'-yl]-6,7-dihydro-8H-cyclopenta[d]-1,2,4-triazolo[1,5-a]pyrimidin-5[9H]-one in 49.5 g (0.022 mole ≈ 30 ml) of phosphorus oxychloride 1.185 g (0.015 mole ≈ 1.2 ml) of pyridine are added, and the reaction mixture is stirred at 80 °C for 2 hours. Then it is cooled, 50 ml of ether are added and the separated crystals are filtered. The crystals are dissolved in about 200 ml of water, the solution is made alkaline with solid sodium hydrogen carbonate and the liberated base is extracted from the aqueous solution twice with 200 ml of chloroform each. The chloroform phases are combined, dried over anhydrous sodium sulfate and evaporated to dryness in vacuo. Thus 21.2 g (96.5 %) of a crystalline product are obtained which is recrystallized from acetonitrile.
M.p.: 259-261 °C .

**Example 42**

**5-Chloro-2-piperidino-6,7-dihydro-8H-cyclopenta[d]--1,2,4-triazolo[1,5-a]pyrimidine**

To a suspension of 19.45 g (0.075 mole) of 2-piperidino-6,7-dihydro-8H-cyclopenta[d]-1,2,4-triazolo[1,5-a]-pyrimidin-5[9H]-one in 49.5 g (0.322 mole ≈ 30 ml) of phosphorus oxychoride 1.185 g (0.015 mole ≈ 1.2 ml) of pyridine are added, and the reaction mixture is stirred at 80 °C for 3 hours. Then it is cooled, 50 ml of ether are added and the separated crystals are filtered. The product is dissolved in about 200 ml of water and the obtained solution is made alkaline with solid sodium hydrogen carbonate. The separated product is filtered and washed successively with water and a slight amount of isopropanol.
Yield: 15.86 g (76.1 %)
M.p.: 124-126 °C.

**Example 43**

**2-[3'-(Dimethylamino)-propyl-amino]-5-chloro-6,7-dihydro-8H--cyclopenta[d]-1,2,4-triazolo[1,5-a]pyrimidine hydrochloride**

A mixture of 13.8 g (0.05 mole) of 2-[3'-(dimethylamino)-propyl-amino]-6,7-dihydro-8H-cyclopenta[d]-1,2,4-triazolo [1,5-a]pyrimidin-5(9H)-one, 32.8 g (0.215 mole ≈ 20 ml) of phosphorus oxychloride and 0.79 g (0.01 mole ≈ 0.8 ml) of pyridine are stirred at 80 °C for 3 hours. Then it is cooled and 30 ml of ether are added. After mixing the ether is decanted, further 30 ml of ether are added to the mixture and the above procedure is repeated. To the residual oil about 50 g of ice are added. When the ice has melted, a solution is obtained which is neutralized with anhydrous sodium hydrogen carbonate. The separated crystals are filtered and washed with a slight amount of water.
Yield: 13.5 g (81.7 %)
M.p.: 270-274 °C .

**Example 44**

**2-[3'-(Dimethylamino)-propyl-amino]-5-chloro-6,7-dihydro-8H--cyclopenta[d]-1,2,4-triazolo[1,5-a]pyrimidine**

13.2 g (0.04 mole) of the hydrochloride salt obtained according to Example 46 are dissolved in 50 ml of water. The solution is rendered alkaline with a 5 % by weight sodium hydroxide solution and extracted with chloroform. The chloroform phase is extracted with water, dried and evaporated.

Yield: 11.05 g (93.7 %)
M.p.: 165-167 °C (after recrystallization from isopropanol).

**Example 45**

**5-Chloro-7-methyl-2-methylthio-1,2,4-triazolo[1,5-a]-pyrimidine**

To a suspension of 49.83 g (0.249 mole) of 7-methyl-2--methylthio-8H-1,2,4-triazolo[1,5-a]pyrimidin-5-one in 153.3 g (1 mole ≈ 93 ml) of phosphorus oxychloride 6.57 g (0.083 mole ≈ 6.7 ml) of pyridine are added and the reaction mixture is stirred for 6 hours while the inner temperature is kept at 80-82 °C. The mixture is allowed to stand overnight, the separated crystals are filtered and washed successively with phosphorus oxychloride, acetone, cold water and acetone again.
Yield: 24.54 g (45 %)
M.p.: 136-138.5 °C .

To the dark red phosphorus oxychloride mother liquor 750 g of crushed ice are added and the product is extracted with 150 ml of chloroform. The chloroform phase is washed with water, dried over sodium sulfate and evaporated to dryness in vacuo. The residue (23.0 g) is suspended in isopropanol, filtered and washed with 2-isopropanol.
Yield: 23.0 g (42.2 %)
M.p.: 136-137 °C .

The products thus obtained are combined and subjected to chromatography on a Kieselgel 60H column (eluent: 8x100 ml of a 2:8 mixture (by volume) of n-hexane and chloroform). Thus 45.4 g (83.3 %) of pale yellow crystals are obtained.
M.p.: 135-135.5 °C (isopropanol)
Total yield: 87.2 % .

**Example 46**

**5-Chloro-7-methyl-2-methylthio-1,2,4-triazolo[1,5-a]-pyrimidine**

22.7 g (0.104 mole) of 7-methyl-2-methylthio-8H-1,2,4-triazolo[1,5-a]pyrimidin-5-one sodium salt are suspended in 49.5 g (0.323 mole ≈ 30 ml) of phosphorus oxychloride under stirring, while the temperature of the reaction mixture raises to 90 °C. The suspension is then cooled to 60 °C, 2.55 g (0.032 mole ≈ 2.6 ml) of pyridine are added and the mixture is stirred at 80 °C for 3 hours. Then it is allowed to cool to 40 °C, diluted with 150 ml of chloroform and poured onto 150 g of crushed ice. The phases are separated, the aqueous phase is extracted with chloroform, the organic phases are combined, washed until neutral with cold diluted sodium hydrogen carbonate solution and water, dried over anhydrous sodium sulfate and evaporated to dryness in vacuo. The residual product (19 g) is suspended in ether, filtered again and washed with ether.
Yield: 17.65 g (79.0 %)
M.p.: 133-134.5 °C.

**Claims**

**Claims for the following Contracting States : AT, BE, CH, DE, DK, FR, GB, IT, LI, LU, MC, NL, PT, SE**

1. 1,2,4-Triazolo[1,5-a]pyrimidine derivatives of the general formula

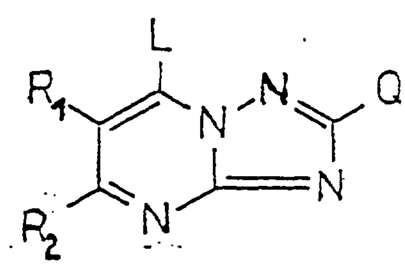

I

wherein

Q represents a morpholino, piperazino or piperidinyl group bound by a nitrogen atom of them to the triazole ring optionally carrying a $C_{1-4}$ alkyl or benzyl substituent; or a group of the formula

$S(O)_p - R_3$ A,

wherein

p stands for 0, 1 or 2 and

$R_3$ denotes straight or branched chained $C_{1-8}$ alkyl, $C_{2-6}$ alkenyl or phenyl-($C_{1-4}$ alkyl), which latter may optionally carry one or more halogen or nitro substituent(s), or phenyl, optionally substituted by one or more nitro and/or trifluoromethyl; or a group of the formula

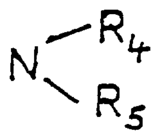 B ,

wherein

$R_4$ and $R_5$ independently each represent hydrogen, straight or branched chained $C_{1-12}$ alkyl, $C_{2-6}$ alkenyl, phenyl--($C_{1-4}$ alkyl) including such having more than 1 phenyl group or di-($C_{1-4}$ alkyl)--amino-($C_{1-6}$ alkyl),

$R_1$ and $R_2$ independently each represent hydrogen or straight or branched chained $C_{1-4}$ alkyl or

$R_1$ and $R_2$ together form a group of the formula

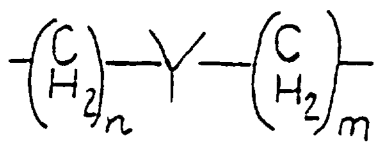 C,

in which latter

Y stands for a $CH_2$ group, a sulfur atom or a group of the formula

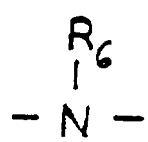 D,

wherein

$R_6$ denotes hydrogen or phenyl-($C_{1-4}$ alkyl),

n and m each represent 0, 1, 2, 3, 4 or 5 and

L represents halogen,

with the provisos that

a) if

L denotes chlorine and

$R_1$ and $R_2$ together form a propylene group,

Q is other than S-benzyl,

b) if

Q represents formula B in which

$R_4$ and $R_5$ mean hydrogen atoms,

L stands for chlorine and
$R_2$ represents a methyl radical

$R_1$ is other than hydrogen and

c) if

Q represents formula A with

p = 0 and
$R_3$ = a benzyl radical,

L means chlorine and
$R_2$ stands for a methyl radical

$R_1$ is other than hydrogen

and their salts.

**2.** 1,2,4-Triazolo[1,5-a]pyrimidine derivatives according to claim 1, characterized in that the halogen(s) which may be represented by L and/or which may be carried by the phenyl-($C_{1-4}$ alkyl) group which may be represented by $R_3$ is/are chlorine and/or bromine.

**3.** 1,2,4-Triazolo[1,5-a]pyrimidine derivatives according to claim 1 or 2, characterized in that the $C_{1-4}$ alkyl substituent which may be carried by the morpholino, piperidino or piperazinyl group which may be represented by Q and/or the alkyl part(s) of the phenyl-($C_{1-4}$ alkyl) group(s) which may be represented by $R_3$ and/or $R_4$ and/or $R_5$ and/or $R_6$ and/or the $C_{1-4}$ alkyl group(s) of the di-($C_{1-4}$ alkyl)-amino part(s) of the di-($C_{1-4}$ alkyl)-amino-($C_{1-6}$ alkyl) group (s) which may be represented by $R_4$ and/or $R_5$ and/or the $C_{1-4}$ alkyl group which may be represented by $R_1$ and/or $R_2$ is/are such one(s) having 1 or 2 carbon atom(s); and/or the $C_{1-8}$ alkyl group which may be represented by $R_3$ is such one having from 1 to 6, particularly from 1 to 3, carbon atom(s); and/or the $C_{2-6}$ alkenyl group(s) which may be represented by $R_3$ or $R_4$ and/or $R_5$ is/are such one(s) having from 2 to 4, particularly 3 carbon atoms and/or the $C_{1-12}$ alkyl group which may be represented by $R_4$ and/or $R_5$ is/are such one[s] having from 1 to 8, particularly 1 to 4, carbon atom(s); or the $C_{1-6}$ alkyl group(s) of the amino-($C_{1-6}$ alkyl) part(s) of the di--($C_{1-4}$ alkyl)-amino-($C_{1-6}$ alkyl) group(s) which may be represented by $R_4$ and/or $R_5$ is/are such one(s) having from 1 to 4 carbon atom (s).

**4.** 5-Chloro-2-methylthio-6,7-dihydro-8H-cyclopenta[d]--1,2,4-triazolo[1,5-a]pyrimidine, 5-chloro-2-methylthio--6,7,8,9-tetrahydro-1,2,4-triazolo[5,1-b]quinazoline, 5-chloro-2-morpholino-6,7,8,9-tetrahydro-1,2,4-triazolo[5,1-b] quinazoline and 5-chloro-7-methyl-2-methylthio--1,2,4-triazolo[1,5-a]-pyrimidine.

**5.** A process for preparing the compounds according to claims 1 to 4, characterized in that a 1,2,4-triazolo[1,5-a] pyrimidine derivative of the general formula

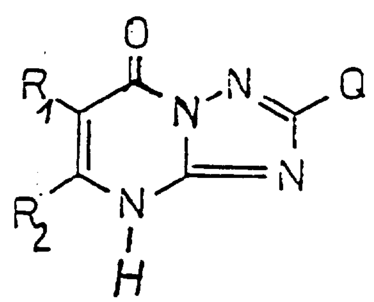

II,

wherein
Q, $R_1$ and $R_2$ are as stated in claims 1 to 3,
or the sodium salt thereof is reacted with a mineral acid halide of the general formula

$$A - X_i \qquad III,$$

wherein

A denotes a mineral acid radical,
X stands for halogen and
i represents 1, 2, 3, 4 or 5

and, if desired, in a manner known per se converting a compound of the general formula I thus obtained into a salt or converting a salt of the compound of the general formula I thus obtained into the free base of formula I or into another salt.

**6.** A process according to claim 5, characterized by using phosphorus oxychloride, phosphorus pentachloride or thionyl chloride as mineral acid halide.

**7.** A process according to claim 5 or 6, characterized by carrying out the reaction in the presence of a catalyst, particularly a tertiary amine base or dimethylformamide.

**8.** A process according to claims 5 to 7, characterized by carrying out the reaction in an inert solvent, particularly in benzene, toluene, xylene, chloroform, dichloroethane and/or chlorobenzene, or in an excess of the mineral acid halide of the general formula III.

**9.** A process according to claims 5 to 8, characterized by carrying out the reaction at a temperature from 20°C to the boiling point of the reaction mixture.

**10.** Medicaments, characterized in that they contain as [an] active ingredient(s) at least one compound according to claims 1 to 4, suitably in admixture with at least one inert solid and/or liquid pharmaceutical carrier and/or diluent and/or additive.

**11.** Use of the compounds according to claims 1 to 4 for preparing medicaments, particularly such having gastric acid secretion inhibiting effect.

**Claims for the following Contracting States : ES, GR**

**1.** A process for preparing 1,2,4-triazolo[1,5-a]pyrimidine derivatives of the general formula

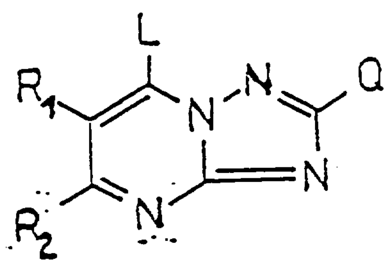

I

wherein

Q represents a morpholino, piperazino or piperidinyl group bound by a nitrogen atom of them to the triazole ring, optionally carrying a $C_{1-4}$ alkyl or benzyl substituent; or a group of the formula

$$S(O)_p - R_3 \qquad A,$$

wherein

p stands for 0, 1 or 2 and
$R_3$ denotes straight or branched chained $C_{1-8}$ alkyl, $C_{2-6}$ alkenyl or phenyl-($C_{1-4}$ alkyl), which latter may optionally carry one or more halogen or nitro substituent(s), or phenyl, optionally substituted by one or more nitro and/or trifluoromethyl; or a group of the formula

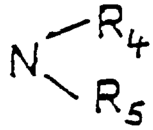 B ,

wherein

$R_4$ and $R_5$ independently each represent hydrogen, straight or branched chained $C_{1-12}$ alkyl, $C_{2-6}$ alkenyl, phenyl--($C_{1-4}$ alkyl) including such having more than 1 phenyl group or di-($C_{1-4}$ alkyl)--amino-($C_{1-6}$ alkyl),

$R_1$ and $R_2$ independently each represent hydrogen or straight or branched chained $C_{1-4}$ alkyl or

$R_1$ and $R_2$ together form a group of the formula

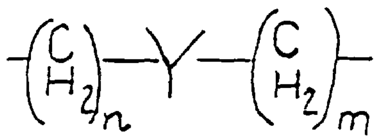 C,

in which latter

Y stands for a $CH_2$ group, a sulfur atom or a group of the formula

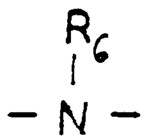 D,

wherein

$R_6$ denotes hydrogen or phenyl-($C_{1-4}$ alkyl),

n and m each represent 0, 1, 2, 3, 4 or 5 and

L represents halogen,

with the provisos that

a) if

L denotes chlorine and
$R_1$ and $R_2$ together form a propylene group,

Q is other than S-benzyl,

b) if

Q represents formula B
in which

$R_4$ and $R_5$ mean hydrogen atoms,

L stands for chlorine and
$R_2$ represents a methyl radical

$R_1$ is other than hydrogen

and

c) if

Q represents formula A with

p = 0 and
$R_3$ = a benzyl radical,

L means chlorine and
$R_2$ stands for a methyl radical

$R_1$ is other than hydrogen

and their salts, characterized in that
a 1,2,4-triazolo[1,5-a]pyrimidine derivative of the general formula

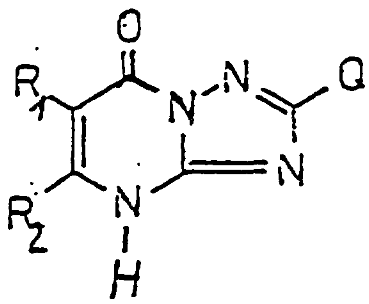

II,

wherein
Q, $R_1$ and $R_2$ are as stated above,.
or the sodium salt thereof is reacted with a mineral acid halide of the general formula

$$A - X_i \qquad III,$$

wherein

A denotes a mineral acid radical,
X stands for halogen and
i represents 1, 2, 3, 4 or 5

and, if desired, in a manner known per se converting a compound of the general formula I thus obtained into a salt or converting a salt of the compound of the general formula I thus obtained into the free base of formula I or into another salt.

**2.** A process according to claim 1, characterized by that 1,2,4-triazolo[1,5-a]pyrimidine derivatives are prepared in which the halogen(s) which may be represented by L and/or which may be carried by the phenyl-($C_{1-4}$ alkyl) group which may be represented by $R_3$ is/are chlorine and/or bromine.

**3.** A process according to claim 1 or 2, characterized by that 1,2,4-triazolo[1,5-a]pyrimidine derivatives are prepared in which the $C_{1-4}$ alkyl substituent which may be carried by the morpholino, piperidino or piperazinyl group which may be represented by Q and/or the alkyl part(s) of the phenyl-($C_{1-4}$ alkyl) group(s) which may be represented by $R_3$ and/or $R_4$ and/or $R_5$ and/or $R_6$ and/or the $C_{1-4}$ alkyl group(s) of the di--($C_{1-4}$ alkyl)-amino part(s) of the di-($C_{1-4}$ alkyl)-amino--($C_{1-6}$ alkyl) group(s) which may be represented by $R_4$ and/or $R_5$ and/or the $C_{1-4}$ alkyl group which may be represented by $R_1$ and/or $R_2$ is/are such one(s) having 1 or 2 carbon atom(s); and/or the $C_{1-8}$ alkyl group which may be represented by $R_3$ is such one having from 1 to 6, particularly from 1 to 3, carbon atom(s); and/or the $C_{2-6}$ alkenyl group(s) which may be represented by $R_3$ or $R_4$ and/or $R_5$ is/are such one(s) having from 2 to 4, particularly 3, carbon atoms and/or the $C_{1-12}$ alkyl group which may be represented by $R_4$ and/or $R_5$ is/are such one(s) having from 1 to 8, particularly 1 to 4, carbon atom(s); or the $C_{1-6}$ alkyl group(s) of the amino--($C_{1-6}$ alkyl) part(s) of the di-($C_{1-4}$ alkyl)-amino-($C_{1-6}$ alkyl) group(s) which may be represented by $R_4$ and/or $R_5$ is/are such one(s) having from 1 to 4 carbon atom(s).

**4.** A process according to claims 1 to 3, characterized by that 5-chloro-2-methylthio-6,7-dihydro-8H-cyclopenta[d]--

1,2,4-triazolo[1,5-a]pyrimidine, 5-chloro-2-methylthio--6,7,8,9-tetrahydro-1,2,4-triazolo[5,1-b]quinazoline, 5-chloro-2-morpholino-6,7,8,9-tetrahydro-1,2,4-triazolo[5,1-b]quinazoline and 5-chloro-7-methyl-2-methylthio-1,2,4-triazolo [1,5-a]-pyrimidine are prepared.

**5.** A process according to claims 1 to 4, characterized by using phosphorus oxychloride, phosphorus pentachloride or thionyl chloride as mineral acid halide.

**6.** A process according to claims 1 to 5, characterized by carrying out the reaction in the presence of a catalyst, particularly a tertiary amine base or dimethylformamide.

**7.** A process according to claims 1 to 6, characterized by carrying out the reaction in an inert solvent, particularly in benzene, toluene, xylene, chloroform, dichloroethane and/or chlorobenzene or in an excess of the mineral acid halide of the general formula III.

**8.** A process according to claims 1 to 7, characterized by carrying out the reaction at a temperature from 20°C to the boiling point of the reaction mixture.

**Patentansprüche**

**Patentansprüche für folgende Vertragsstaaten : AT, BE, CH, DE, DK, FR, GB, IT, LI, LU, MC, NL, PT, SE**

**1.** 1,2,4-Triazolo[1,5-a]pyrimidin-Derivate der allgemeinen Formel (I),

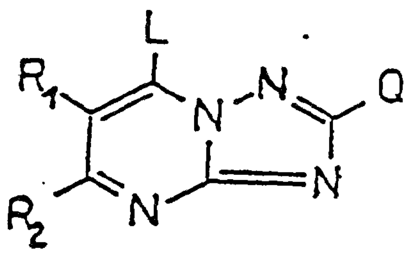

(I)

worin

Q Morpholino-, Piperazino- or Piperidinylgruppe bedeutet, die durch ein Stickstoffatom dem Triazolring gebunden ist und gegebenenfalls einen $C_{1-4}$ Alkyl- oder Benzyl-Substituenten trägt;
oder eine Gruppe der Formel A bedeutet,

$$S(O)_p\text{-}R_3 \quad (A)$$

worin

p 0, 1 oder 2 ist und

$R_3$ für eine gerade oer abgezweigte $C_{1-8}$ Alkylgruppe, $C_{2-6}$ Alkenylgruppe oder Phenyl-($C_{1-4}$ alkyl)-gruppe steht, wobei die letztere gegebenenfalls einen oder mehrere Halogen- oder Nitro-Substituenten tragen kann, oder für eine gegebenenfalls durch ein (e) oder mehrere Stickstoffatom(e) und/oder Trifluormethylgruppe(n) substituierte Phenylgruppe bedeutet;
oder für eine Gruppe der Formel B steht,

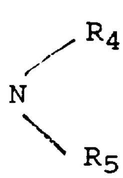

(B)

worin

$R_4$ und $R_5$ unabhängig voneinander Wasserstoff, gerade oder abgezweigte $C_{1-12}$ Alkyl, $C_{2-6}$ Alkenyl, Phenyl-($C_{1-4}$ alkyl) inbegriffen solche, die mehr als eine Phenylgruppe enthal-

ten, oder Di-($C_{1-4}$ alkyl)-amino-($C_{1-6}$ alkyl)-gruppe bedeuten,

$R_1$ und $R_2$ unabhängig voneinander Wasserstoff oder gerade oder abgezweigte $C_{1-4}$ Alkylgruppen bedeuten oder

$R_1$ und $R_2$ zusammen eine Gruppe der Formel C bedeuten,

$$-(CH_2)_n - Y - (CH_2)_m - \qquad (C)$$

wobei in der letzteren Gruppe

Y für eine $CH_2$-Gruppe, ein Schwefelatom oder eine Gruppe der Formel D steht,

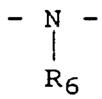

(D)

worin

$R_6$ Wasserstoff oder eine Phenyl-($C_{1-4}$ alkyl)-gruppe bedeutet,

n und m 0, 1, 2, 3, 4 oder 5 bedeuten und

L Halogen bedeutet,

mit der Einschränkung, dass

a) falls

L Chlor bedeutet und

$R_1$ und $R_2$ zusammen eine Propylengruppe bedeuten,

Q eine andere Bedeutung als S-Benzyl hat,

b) falls

Q eine Gruppe der Formel B bedeutet, worin

$R_4$ und $R_5$ Wasserstoffatome bedeuten,

L für Chlor steht und

$R_2$ eine Methylgruppe bedeutet,

$R_1$ eine von Wasserstoff abweichende Bedeutung hat,

c) falls

Q eine Gruppe der Formel A bedeutet, worin

p = 0 und

$R_3$ = Benzylgruppe,

L Chlor ist und

$R_2$ eine Methylgruppe bedeutet,

$R_1$ eine von Wasserstoff abweichende Bedeutung hat,

und ihre Salze.

**2.** 1,2,4-Triazolo[1,5-a]pyrimidin-Derivate nach Patentanspruch 1, dadurch gekennzeichnet, dass das/die Halogen (e) der Formel L, das/die durch die Phenyl-($C_{1-4}$ alkyl)-gruppe der Formel $R_3$ getragen werden kann/können, Chlor und/oder Brom sein kann/können.

**3.** 1,2,4-Triazolo[1,5-a]pyrimidin-Derivate nach Patentanspruch 1 oder 2, dadurch gekennzeichnet, dass der $C_{1-4}$ Alkyl-Substituent, der durch die Morpholino-, Piperidino- oder Piperazinylgruppe der Formel Q getragen werden kann, und/oder der/die Alkylteil(e) der Phenyl-($C_{1-4}$ alkyl)-gruppe(n) der Formel $R_3$ und/oder $R_4$ und/oder $R_5$ und/oder die $C_{1-4}$ Alkylgruppe(n) des/der di-($C_{1-4}$ alkyl)-amino-Teile(s) der Di--($C_{1-4}$ alkyl)-amino-($C_{1-6}$ alkyl)-gruppe(n) der Formel $R_4$ und/oder $R_5$ und/oder die $C_{1-4}$ Alkylgruppe der Formel $R_1$ und/oder $R_2$ 1 oder 2 Kohlenstoffatom(e) hat/haben; und/oder die $C_{1-8}$ Alkylgruppe der Formel $R_3$ 1 bis 6, insbesondere 1 bis 3, Kohlenstoffatom (e) aufweist; und/oder die $C_{2-6}$ Alkenylgruppe(n) der Formel $R_3$ oder $R_4$ und/oder $R_5$ 2 bis 4, insbesondere 3, Kohlenstoffatome hat/haben und/oder die $C_{1-12}$ Alkylgruppe der Formel $R_4$ und/oder $R_5$ 1 bis 8, insbesondere 1 bis 4, Kohlenstoffatom(e) aufweist; oder die $C_{1-6}$ Alkylgruppe(n) des/der Amino-($C_{1-6}$ alkyl)-Teile(s) der Di-($C_{1-4}$ alkyl)-amino-($C_{1-6}$ alkyl)-gruppe(n) der Formel $R_4$ und/oder $R_5$ 1 bis 4 Kohlenstoffatom(e) hat/haben.

**4.** 5-Chlor-2-methylthio-6,7-dihydro-8H-ciklopenta[d]--1,2,4-triazolo[1,5-a]-pyrimidin, 5-Chlor-2-methylthio--6,7,8,9-tetrahydro-1,2,4-triazolo[5,1-b]chinazolin, 5-Chlor--2-morpholino-6,7,8,9-tetrahydro-1,2,4-triazolo[5,1-b]chinazolin und 5-Chlor-7-methyl-2-methylthio-1,2,4-triazolo[1,5-a]pyrimidin.

**5.** Verfahren zur Herstellung der Verbindungen nach den Patentansprüchen 1 bis 4, dadurch gekennzeichnet, dass ein 1,2,4-Triazolo[1,5-a]pyirimidin-Derivat der allgemeinen Formel (II),

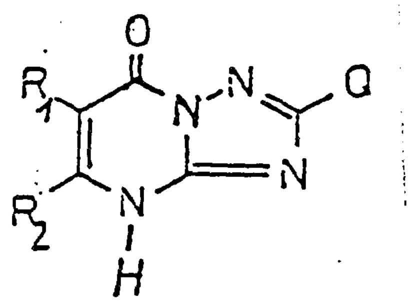

(II)

worin

Q, $R_1$ und $R_2$ die in den Patentansprüchen 1 bis 3 angegebenen Bedeutungen haben,

oder sein Natriumsalz mit einer Mineralsäurehalogenid der allgemeinen Formel (III),

$$A - X_i \quad \text{(III)}$$

worin

A ein Mineralsäureradikal bedeutet,
X für Halogen steht und
i 1, 2, 3, 4 oder 5 bedeutet,

umgesetzt wird, und gegebenenfalls eine derart erhaltene Verbindung der allgemeinen Formel (I) in ein Salz in an sich bekannter Weise übergeführt oder ein auf diese Weise erhaltenes Salz der Verbindung der allgemeinen Formel (I) in eine freie Base der Formel (I) oder ein anderes Salz übergeführt wird.

**6.** Verfahren nach Patentanspruch 5, dadurch gekennzeichnet, dass Phosphoroxychlorid, Phosphorpentachlorid oder Thionylchlorid als Mineralsäurehalogenid verwendet wird.

**7.** Verfahren nach Patentanspruch 5 oder 6, dadurch gekennzeichnet, dass die Umsetzung in Anwesenheit eines Katalysators, insbesondere einer tertiären Aminbase oder von Dimethylformamid durchgeführt wird.

**8.** Verfahren nach den Patentanspüchen 5 bis 7, dadurch gekennzeichnet, dass die Umsetzung in einem inerten Lösungsmittel, insbesondere in Benzol, Toluol, Xylol, Chloroform, Dichlorethan und/oder Chlorbenzol, oder in einem Überschuss des Mineralsäurehalogenids der allgemeinen Formel (III) durchgeführt wird.

**9.** Verfahren nach den Patentansprüchen 5 bis 8, dadurch gekennzeichnet, dass die Umsetzung bei einer Temperatur zwischen 20 °C und dem Siedepunkt des Reaktionsgemisches durchgeführt wird.

**10.** Medizine, dadurch gekennzeichnet, dass sie mindestens eine Verbindung nach den Patentansprüchen 1 bis 4 als aktive Komponente(n) enthalten, vorzugsweise unter Beimischung von mindestens einem inerten festen und/oder flüssigen pharmazeutischen Trägerstoff und/oder Verdünnungsmittel und/oder Zusatzstoff.

**11.** Verwendung der Verbindungen nach den Patentansprüchen 1 bis 4 zur Herstellung von Medizinen, besonders von solchen, die eine die Sekretion der Magensäure inhibierende Wirkung aufweisen.

**Patentansprüche für folgende Vertragstaaten : ES, GR**

**1.** Verfahren zur Herstellung von 1,2,4-Triazolo[1,5-a]-pyrimidin-Derivaten der allgemeinen Formel (I),

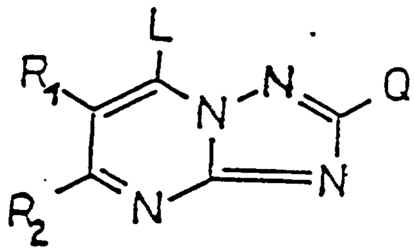

(I)

worin

Q Morpholino-, Piperazino- or Piperidinylgruppe bedeutet, die durch ein Stickstoffatom dem Triazolring gebunden ist und gegebenenfalls einen $C_{1-4}$ Alkyl- oder Benzyl-Substituenten trägt; oder eine Gruppe der Formel A bedeutet,

$$S(O)_p\text{-}R_3 \qquad (A)$$

worin

p 0, 1 oder 2 ist und

$R_3$ für eine gerade oer abgezweigte $C_{1-8}$ Alkylgruppe, $C_{2-6}$ Alkenylgruppe oder Phenyl-($C_{1-4}$ alkyl)-gruppe steht, wobei die letztere gegebenenfalls einen oder mehreren Halogen- oder Nitro-Substituenten tragen kann, oder für eine gegebenenfalls durch ein (e) oder mehrere Stickstoffatom(e) und/oder Trifluormethylgruppe(n) substituierte Phenylgruppe bedeutet;
oder für eine Gruppe der Formel B steht,

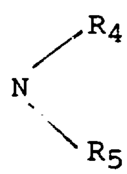

(B)

worin

$R_4$ und $R_5$ unabhängig voneinander Wasserstoff, gerade oder abgezweigte $C_{1-12}$ Alkyl, $C_{2-6}$ Alkenyl, Phenyl-($C_{1-4}$ alkyl) inbegriffen solche, die mehr als eine Phenylgruppe enthalten, oder Di-($C_{1-4}$ alkyl)-amino-($C_{1-6}$ alkyl)-gruppe bedeuten,

$R_1$ und $R_2$ unabhängig voneinander Wasserstoff oder gerade oder abgezweigte $C_{1-4}$ Alkylgruppen bedeuten oder

$R_1$ und $R_2$ zusammen eine Gruppe der Formel C bedeuten,

$$-(CH_2)_n - Y - (CH_2)_m - \qquad (C)$$

wobei in der letzteren Gruppe

Y für eine $CH_2$-Gruppe, ein Schwefelatom oder eine Gruppe der Formel D steht,

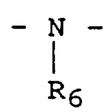

(D)

worin

$R_6$ Wasserstoff oder eine Phenyl--($C_{1-4}$ alkyl)-gruppe bedeutet,

n und m 0, 1, 2, 3, 4 oder 5 bedeuten und

L Wasserstoffatom bedeutet,

mit der Einschränkung, dass

a) falls

L Chlor bedeutet und
$R_1$ und $R_2$ zusammen eine Propylengruppe bedeuten,

Q eine andere Bedeutung als S-Benzyl hat,

b) falls

Q eine Gruppe der Formel B bedeutet, worin

$R_4$ und $R_5$ Wasserstoffatome bedeuten,

L für Chlor steht und
$R_2$ eine Methylgruppe bedeutet,

$R_1$ eine von Wasserstoff abweichende Bedeutung hat,

c) falls

Q eine Gruppe der Formel A bedeutet, worin

p = 0 und
$R_3$ = Benzylgruppe,

L Chlor ist und
$R_2$ eine Methylgruppe bedeutet,

$R_1$ eine von Wasserstoff abweichende Bedeutung hat,

und ihre Salze, dadurch gekennzeichnet, dass ein 1,2,4--Triazolo[1,5-a]pyirimidin-Derivat der allgemeinen Formel (II),

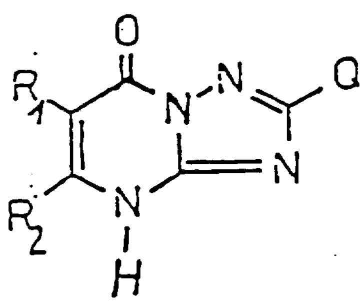

(II)

worin
Q, $R_1$ und $R_2$ die obigen Bedeutungen haben,
oder sein Natriumsalz mit einem Mineralsäurehalogenid der allgemeinen Formel (III),

$$A - X_i \quad (III)$$

worin

A ein Mineralsäureradikal bedeutet,
X für Halogen steht und
i 1, 2, 3, 4 oder 5 bedeutet,

umgesetzt wird, und gegebenenfalls eine Verbindung der allgemeinen Formel (I) in an sich bekannter Weise in

ein Salz übergeführt oder ein Salz der auf diese Weise erhaltenen Verbindung der allgemeinen Formel (I) in eine freie Base der Formel (I) oder ein anderes Salz übergeführt wird.

2. Verfahren nach Patentanspruch 1, dadurch gekennzeichnet, dass 1,2,4-Triazolo[1,5-a]pyrimidin-Derivate hergestellt werden, worin das/die Halogen(e) der Formel L, das/die durch die Phenyl-($C_{1-4}$ alkyl)-gruppe der Formel $R_3$ getragen werden kann/können, Chlor oder Brom ist/sind.

3. Verfahren nach Patentanspruch 1 oder 2, dadurch gekennzeichnet, dass 1,2,4-Triazolo[1,5-a]pyrimidin-Derivate hergestellt werden, in welchen der $C_{1-4}$ Alkylsubstituent, der durch die Morpholino-, Piperidino- oder Piperazinylgruppe der Formel Q getragen werden kann und/oder der/die Alkylteil(e) der Phenyl-($C_{1-4}$ alkyl)-gruppe(n) der Formel $R_3$ und/oder $R_4$ und/oder $R_5$ und/oder $R_6$ und/oder die $C_{1-4}$ Alkylgruppe(n) des/der di-($C_{1-4}$ alkyl)-amino-Teile(s) der Di-($C_{1-4}$ alkyl)-amino-($C_{1-6}$ alkyl)-gruppe(n) der Formel $R_4$ und/oder $R_5$ und/oder die $C_{1-4}$ Alkylgruppe der Formel $R_1$ und/oder $R_2$ 1 oder 2 Kohlenstoffatom(e) hat/haben; und/oder die $C_{1-8}$ Alkylgruppe der Formel $R_3$ 1 bis 6, insbesondere 1 bis 3, Kohlenstoffatom(e) aufweist; und/oder die $C_{2-6}$ Alkenylgruppe(n) der Formel $R_3$ oder $R_4$ und/oder $R_5$ 2 bis 4, insbesondere 3, Kohlenstoffatome hat/haben und/oder die $C_{1-12}$ Alkylgruppe der Formel $R_4$ und/oder $R_5$ 1 bis 8, insbesondere 1 bis 4, Kohlenstoffatom(e) aufweist; oder die $C_{1-6}$ Alkylgruppe(n) des/der Amino-($C_{1-6}$ alkyl)-Teile(s) der Di-($C_{1-4}$ alkyl)-amino-($C_{1-6}$ alkyl)-gruppe(n) der Formel $R_4$ und/oder $R_5$ 1 bis 4 Kohlenstoffatom(e) hat/haben.

4. Verfahren nach den Patentansprüchen 1 bis 3, dadurch gekennzeichnet, dass 5-Chlor-2-methylthio-6,7-dihydro-8H-cyklopenta[d]-1,2,4-triazolo[1,5-a]pyrimidin, 5-Chlor-2-methylthio-6,7,8,9-tetrahydro-1,2,4-triazolo[5,1-b]chinazolin, 5-Chlor-2-morpholino-6,7,8,9-tetrahydro-1,2,4-triazolo[5,1-b]chinazolin und 5-Chlor-7-methyl-2-methylthio-1,2,4-triazolo[1,5-a]pyrimidin hergestellt werden.

5. Verfahren nach den Patentansprüchen 1 bis 4, dadurch gekennzeichnet, dass Phosphoroxychlorid, Phosphorpentachlorid oder Thionylchlorid als Mineralsäurehalogenid verwendet wird.

6. Verfahren nach den Patentansprüchen 1 bis 5, dadurch gekennzeichnet, dass die Umsetzung in Gegenwart eines Katalysators, insbesondere einer tertiären Aminbase oder Dimethylformamid, durchgeführt wird.

7. Verfahren nach den Patentansprüchen 1 bis 6, dadurch gekennzeichnet, dass die Umsetzung in einem inerten Lösungsmittel, insbesondere in Benzol, Toluol, Xylol, Chloroform, Dichlorethan und/oder Chlorbenzol oder in einem Überschuss des Mineralsäurehalogenids der allgemeinen Formel (III) durchgeführt wird.

8. Verfahren nach den Patentansprüchen 1 bis 7, dadurch gekennzeichnet, dass die Umsetzung bei einer Temperatur zwischen 20 °C und dem Siedepunkt des Reaktionsgemisches durchgeführt wird.

**Revendications**

**Revendications pour les Etats contractants suivants : AT, BE, CH, DE, DK, FR, GB, IT, LI, LU, MC, NL, PT, SE**

1. Dérivés de 1,2,4 - triazolo[1,5 - a]pyrimidine de formule générale (1)

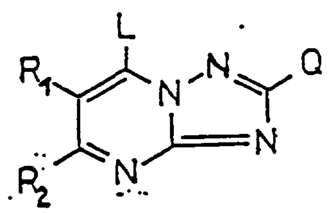

I

dans laquelle

Q représente un groupe morpholino, pipérazino ou pipéridinyle lié par un atome d'azote de ceux-ci à l'anneau de triazole, et éventuellement portant un substituant alkyle ayant de 1 à 4 atomes de carbone ou benzyle; ou un groupe de formule A

$S(O)_p-R_3$

dans laquelle

p est 0, 1 ou 2 et

$R_3$ représente un reste alkyle linéaire ou ramifié, ayant de 1 à 8 atomes de carbone, alkényle ayant de 2 à 6 atomes de carbone ou phényl-(alkyl en C $_{1-4}$), ce dernier pouvant porter éventuellement un ou plusieurs substituant(s) halogène ou nitro; ou phényle éventuellement substitué par un ou plusieurs reste(s) nitro et/ou trifluorométhyle;
ou un groupe de la formule B,

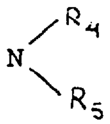

(B)

dans laquelle

$R_4$ et $R_5$ représentent indépendamment l'un de l'autre de l'hydrogène, un reste alkyle linéaire ou ramifié ayant de 1 à 12 atomes de carbone, alkényle ayant de 2 à 6 atomes de carbone, phényl-(alkyl en C $_{1-4}$) comportant ceux qui ont plus d'un groupe phényle, ou di-(alkyl en C 1-4)-amino-(alkyl en C $_{1-6}$),

$R_1$ et $R_2$ représentent indépendamment l'un de l'autre un atome d'hydrogène ou un reste alkyle linéaire ou ramifié, ayant de 1 à 4 atomes de carbone où

$R_1$ et $R_2$ forment ensemble un groupe de formule C,

$$-(CH_2)_n-Y-(CH_2)_m- \quad (C)$$

où dans ce dernier

Y signifie un groupe $CH_2$, un atome de soufre ou un groupe de formule D

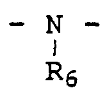

(D)

dans laquelle

$R_6$ représente un atome d'hydrogène ou un reste phényl-(alkyl en C 1-4)

n et m représentent chacun 0, 1, 2, 3, 4 ou 5 et

L représente un halogène,

avec la condition supplémentaire que

a) si

L signifie un atome de chlore et

$R_1$ et $R_2$ forment ensemble un groupe propylène,

Q est autre qu'un reste S-benzyle,

b) si

Q représente la formule B, dans laquelle

$R_1$ et $R_5$représentent des atomes d'hydrogène,

L représente un atome de chlore et

$R_2$ signifie un radical méthyle,

$R_1$ est autre qu'un atome d'hydrogène et

c) si

Q représente la formule A, où

p = 0 et
$R_3$ = un radical benzyle,

L signifie un atome de chlore et
$R_2$ représente un radical méthyle,
$R_1$ est autre qu'un atome d'hydrogène,

et leurs sels.

**2.** Dérivés de 1,2,4-triazolo[1,5-a]pyrimidine selon la revendication 1, caractérisés en ce que le/les halogène(s) de formule L et/ou pouvant être porté(s) par le groupe phényl-(alkyl en $C_{1-4}$) de formule $R_3$ est/sont des atomes de chlore et/ou de brome.

**3.** Dérivés de 1,2,4 - triazolo[1,5 - a]pyrimidine selon la revendication 1 ou 2, caractérisés en ce que le substituant alkyle ayant de 1 à 4 atomes de carbone pouvant être porté par le groupe morpholino, pipéridino ou pipérazinyle de formule Q et/ou la/les partie(s) alkyliques du/des groupe(s) phényl-(alkyl en $C_{1-4}$) de formule $R_3$ et/ou $R_4$ et $R_5$ et/ou $R_6$ et/ou du/des groupe(s) alkyle(s) ayant de 1 à 4 atomes de carbone de la/des partie(s) di-(alkyl en $C_{1-4}$)-aminique(s) du/des groupe(s) di-(alkyl en $C_{1-4}$)-amino-(alkyl en $C_{1-6}$) de formule $R_4$ et/ou $R_5$ et/ou le groupe alkyle ayant de 1 à 4 atomes de carbone de formule $R_1$ et/ou $R_2$ a/ont 1 ou 2 atome(s) de carbone; et/ou le groupe alkyle ayant de 1 à 8 atomes de carbone de formule $R_3$ a 1 à 6 et spécialement 1 à 3 atome(s) de carbone; et/ou le(s) groupe(s) alkényle(s) ayant de 2 à 6 atomes de carbone de formule $R_3$ ou $R_4$ et/ou $R_5$ a/ont 2 à 4 et spécialement 3 atomes de carbone et/ou le groupe alkyle ayant de 1 à 12 atomes de carbone de formule $R_4$ et/ou $R_5$ a 1 à 8 et spécialement 1 à 4 atome(s) de carbone; ou le(s) groupe(s) alkyle(s) ayant de 1 à 6 atome (s) de carbone de la (des) partie(s) amino-(alkyl en $C_{1-4}$) de la (des) groupe(s) di-(alkyl en $C_{1-4}$)-amino-(alkyl en $C_{1-6}$) de formule $R_4$ et/ou $R_5$ a/ont 1 à 4 atome(s) de carbone.

**4.** 5-Chlore-2-méthylthio-6,7-dihydro-8H-cyclopenta[d]1,2,4-triazolo[1,5-a]pyrimidine, 5-chlore-2-méthylthio-6,7,8,9-tétrahydro-1,2,4-triazolo]5,1-b]quinazoline, 5-Chlore-2-morpholino-6,7-dihydro-8H-cyclopenta[d]1,2,4-triazolo[1,5-a]pyrimidine, 5-chlore-2-méthylthio-6,7,8,9-tétrahydro-1,2,4-1,2,4-triazolo[1,5-a]pyrimidine, 5-chlore-2-morpholino-6,7,8,9-tétrahydro-1,2,4-triazolo[5,1-b]quinazoline.

**5.** Procédé pour la préparation des composés selon les revendications 1 à 4, caractérisé en ce qu'un dérivé de 1,2,4 - triazolo[1,5 - a]pyrimidine de formule générale II

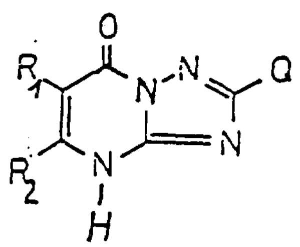

(II)

dans laquelle
Q, $R_1$ et $R_2$ ont une signification selon les revendications 1 à 3;
ou leur sel de sodium est mis en réaction avec un halogénure d'acide minéral de formule générale (III)

$$A - X_i \quad \text{(III)}$$

dans laquelle

A représente un radical d'un acide minéral,
X est un halogénure et
i représente 1, 2, 3, 4 ou 5,

et, si cela est désiré, un composé de formule générale (I) ainsi obtenu est transformé en un sel ou un sel du composé de formule (I) ainsi obtenu est transformé en la base libre de formule (I) ou en un autre sel.

**6.** Procédé selon la revendication 5, caractérisé en ce que de l'oxychlorure de phosphore, du pentachlorure de phosphore ou du chlorure de thionyle est utilisé comme halogénure d'un acide minéral,

**7.** Procédé selon la revendication 5 ou 6, caractérisé en ce que la réaction est réalisée en la présence d'un catalyseur, spécialement d'une base aminique tertiaire ou de formamide diméthylique.

**8.** Procédé selon les revendications 5 à 7, caractérisé en ce que la réaction est réalisée dans un solvant inerte, particulièrement dans du benzène, du toluène, du xylène, du chloroforme, du dichloroétane et/ou du chlorobenzène, ou dans l'excès de l'halogénure d'acide minéral de formule générale (III);

**9.** Procédé selon les revendications de 5 à 8, caractérisé en ce que la réaction est réalisée à une température comprise entre 20 Cϕ et le point d'ébullition du mélange réactionnel.

**10.** Médicaments caractérisés en ce qu'ils contiennent comme ingrédient(s) actif(s) au moins un composé selon les revendications 1 à 4, de préférence mélangés à au moins un support inerte solide et/ou liquide pharmaceutique et/ou un diluant et/ou un additif.

**11.** Utilisation des composés selon les revendications 1 à 4 pour préparer des médicaments, en particulier ceux ayant un effet inhibant la sécrétion de l'acide gastrique.

**Revendications pour les Etats contractants suivants : ES, GR**

**1.** Procédé pour la préparation de dérivés de 1,2,4 - triazolo[1,5 - a]pyrimidine de formule générale (I)

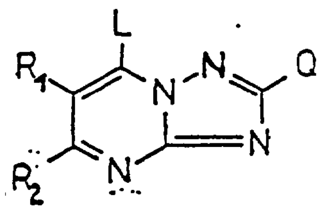

I

dans laquelle

Q représente un groupe morpholino, pipérazino ou pipéridinyle lié par un atome d'azote de ceux-ci à l'anneau de triazole, et éventuellement portant un substituant alkyle ayant de 1 à 4 atomes de carbone ou benzyle;
ou un groupe de formule A

$$S(O)_p\text{-}R3$$

dans laquelle

p est 0,1 ou 2 et

$R_3$ représente un reste alkyle linéaire ou ramifié, ayant de 1 à 8 atomes de carbone, alkényl ayant de 2 à 6 atomes de carbone ou phényl-(alkyl en C 1-4), ce dernier pouvant porter éventuellement un ou plusieurs substituant(s) halogène ou nitro; ou phényle éventuellement substitué par un ou plusieurs reste(s) nitro et/ou trifluorométhyle;
ou un groupe de la formule B,

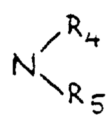

dans laquelle

$R_4$ et $R_5$ représentent indépendamment l'un de l'autre un reste alkyle linéaire ou ramifié, ayant de 1 à 12 atomes de carbone, alkényle ayant de 2 à 6 atomes de carbone, phényl-(alkyl en C 1-4) comportant ceux qui ont plus d'un groupe phényle ou di-(alkyl en C 1-4)-amino-(alkyl en C 1-4),

$R_1$ et $R_2$ représentent indépendamment l'un de l'autre un atome d'hydrogène ou un reste alkyle linéaire ou ramifié, ayant de 1 à 4 atomes de carbone ou

$R_1$ et $R_2$ forment ensemble un groupe de formule C,

$- (CH_2) - Y - (CH_2) -$ (C)

où dans ce dernier

Y signifie un groupe $CH_2$, un atome de soufre ou un groupe de formule D

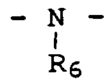

(D)

dans laquelle

$R_6$ représente un atome d'hydrogène ou un reste phényl-(alkyl en C 1-4)
n et m représentent chacun 0, 1, 2, 3, 4 ou 5 et

L représente un halogène,

avec la condition supplémentaire que

a) si

L signifie un atome de chlore et
$R_1$ et $R_2$ forment ensemble un groupe propylène,

Q est autre qu'un reste S-benzyle,

b) si

Q représente la formule B, dans laquelle

$R_1$ et $R_5$ représentent des atomes d'hydrogène,
L représente un atome de chlore et
$R_2$ signifie un radical méthyle,
$R_1$ est autre qu'un atome d'hydrogène et

c) si

Q représente la formule A,
où

p = 0 et
$R_3$ = un radical benzyle,

L signifie un atome de chlore et
$R_2$ représente un radical méthyle,
$R_1$ est autre qu'un atome d'hydrogène,

et leurs sels, caractérisé en ce que l'on fait réagir un dérivé de 1,2,4-triazolo[1,5-a]pyrimidine de formule générale (2)

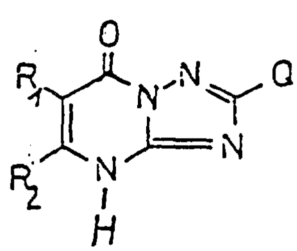

(II)

dans laquelle
Q, $R_1$ et $R_2$ ont les significations ci-dessus,
ou son sel de sodium avec l'halogénure d'un acide minéral de formule générale (III),

A-Xi (III)

où

A est le radical d'un acide minéral
X signifie un halogénure et
i signifie 1, 2, 3, 4 ou 5

le cas échéant on transforme un composé de fomule générale (I), d'une manière connue en soi, en un sel ou l'on transforme un sel du composé ainsi obtenu de formule générale (I) en une base libre de formule (I) ou en un autre sel.

**2.** Procédé selon la revendication 1, caractérisé en ce que l'on prépare des dérivés de 1,2,4 - triazolo[1,5 - a]pyrimidine dans lesquels le(s) halogénure(s) de formule L pouvant être porté(s) par le groupe phényl-(alkyl en C 1-4) de formule $R_3$, est/sont du chlore ou du brome.

**3.** Procédé suivant la revendication 1 ou 2, caractérisé en ce que l'on prépare des dérivés de 1,2,4 - triazolo[1,5 - a] pyrimidine, dans lesquels le substituant alkyle ayant de 1 à 4 atomes de carbone pouvant être porté par le groupe morpholino, pipéridino ou pipérazinyle de formule Q et/ou la/les partie(s) alkyliques du/des groupe(s) phényl-(alkyl en C 1-4) de formule $R_3$ et/ou $R_4$ et $R_5$ et/ou $R_6$ et/ou du/des groupe(s) alkyle(s) ayant de 1 à 4 atomes de carbone de la/les partie(s) di-(alkyl en C 1-4)-aminique(s) du/des groupe(s) di-(alkyl en C 1-4)-amino-(alkyl en C 1-4) de formule $R_4$ et/ou $R_5$ et/ou le groupe alkyle ayant de 1 à 4 atomes de carbone de formule $R_1$ et/ou $R_2$ a/ont 1 ou 2 atome(s) de carbone; et/ou le groupe alkyle ayant de 1 à 8 atomes de carbone de formule 3 a 1 à 6 et en partyiculier 1 à 3 atome(s) de carbone; et/ou le(s) groupe(s) alkényle(s) ayant de 2 à 6 atomes de carbone de fromule $R_3$ ou $R_4$ et/ou $R_5$ a/ont 2 à 4 et spécialement 3 atomes de carbone et/ou le groupe alkyle ayant de 1 à 12 atomes de carbone de formule $R_4$ et/ou $R_5$ a 1 à 8 et en particulier 1 à 4 atome(s) de carbone; ou le(s) groupe(s) alkyle(s) ayant de 1 à 6 atome(s) de carbone de la (des) partie(s) amino-(alkyl en C 1-6) du (des) groupe(s) di-(alkyl en C 1-4)-amino-(alkyl en C 1-6) de formule $R_4$ et/ou $R_5$ a/ont 1 à 4 atome(s) de carbone.

**4.** Procédé suivant les revendications 1 à 3, caractérisé en ce que l'on prépare de la 5-chloro-2-méthylthio-6,7-dihydro-8H-cyclopenta[d]1,2,4-triazolo[1,5-a]pyrimidine, de la 5-chloro-2-méthylthio-6,7,8,9-tétrahydro-1,2,4-triazolo]5,1-b]quinazoline, de la 5-chloro-7-méthyl-2-méthylthio-1,2,4-triazolo[1,5-a]pyrimidine et de la 5-chloro-2-morpholino-6,7,8,9-tétrahydro-1,2,4-triazolo[5,1-b]quinazoline.

**5.** Procédé suivant les revendications 1 à 4, caractérisé en ce que l'on met en oeuvre de l'oxychlorure de phosphore, du pentachlorure de phosphore ou du chlorure de thionyle comme halogénure d'un acide minéral.

**6.** Procédé suivant les revendications 1 à 5, caractérisé en ce que l'on réalise la réaction en la présence d'un catalyseur, spécialement d'une base aminique tertiaire ou de formamide diméthylique.

**7.** Procédé suivant les revendicatioons 1 à 6, caractérisé en ce que l'on réalise la réaction dans un solvant inerte, spécialement dans du benzène, du tholuène, du xylène, du chlorophorme, du dichloroéthane et/ou du chlorobenzène ou dans l'excès de l'halogénure d'un acide minéral de formule générale (III).

**8.** Procédé suivant les revendications 1 à 7, caractérisé en ce que l'on réalise la réaction à une température entre 20 Cϕ et le point d'ébullition du mélange réactionnel.